# EUROPÄISCHE PATENTSCHRIFT

(11) **EP 1 454 136 B1**
(45) Veröffentlichungstag und Bekanntmachung des Hinweises auf die Patenterteilung: **29.04.2009**
(21) Anmeldenummer: 02792793.8
(22) Anmeldetag: 22.11.2002
(51) Int. Cl.: G01N 33/50, G01N 33/68

(54) **VERFAHREN UND MITTEL ZUR BEEINFLUSSUNG VON INTERZELLULÄRER KOMMUNIKATION UND INTERZELLULÄREM ORGANELLENTRANSPORT**
METHODS AND MEANS FOR INFLUENCING INTRACELLULAR COMMUNICATION AND INTRACELLULAR ORGANELLE TRANSPORT
PROCEDES ET AGENTS PERMETTANT D'INFLUENCER LA COMMUNICATION INTERCELLULAIRE ET LE TRANSPORT INTERCELLULAIRE DES ORGANITES

(30) Priorität: 23.11.2001 DE 10157475
(43) Veröffentlichungstag der Anmeldung: 08.09.2004
(60) Teilanmeldung: 08171885.0
(73) Patentinhaber: Stiftelsen Universitetsforskning Bergen, 5020 Bergen (NO)
(72) Erfinder: GERDES, Hans-Hermann, 5009 Bergen (NO); RUSTOM, Amin, c/o Universität Heidelberg, 69120 Heidelberg (DE)
(74) Vertreter: Benedum, Ulrich Max
(86) Internationale Anmeldenummer: PCT/EP2002/013140
(87) Internationale Veröffentlichungsnummer: WO 2003/044524

(56) Entgegenhaltungen:
- WO-A-98/02186
- ELLIOTT G ET AL: "INTERCELLULAR TRAFFICKING AND PROTEIN DELIVERY BY A HERPESVIRUS STRUCTURAL PROTEIN" CELL, CELL PRESS, CAMBRIDGE, NA, US, Bd. 88, 24. Januar 1997 (1997-01-24), Seiten 223-233, XP000961185 ISSN: 0092-8674 in der Anmeldung erwähnt
- GUERINEAU NATHALIE C ET AL: "Organotypic cultures of the rat anterior pituitary: Morphology, physiology and cell-to-cell communication" JOURNAL OF NEUROSCIENCE METHODS, Bd. 73, Nr. 2, 1997, Seiten 169-176, XP009019875 ISSN: 0165-0270

## Beschreibung

### Gebiet der Erfindung

Die Erfindung betrifft pharmazeutische und physikalische Mittel zur Beinflussung von Zellinteraktion, interzellulärem Transport und interzellulärer Kommunikation sowie Verfahren und Mittel zu deren Untersuchung.

### Hintergrund der Erfindung

Die Zellen in multizellulären Organismen und Verbänden tauschen sich nur auf vorgegebenen Wegen aus. Die Kommunikation über kurze Strecken erfolgt soweit bekannt über Gap-Junctions, Plasmodesmata oder synaptische Verbindungen (chemische Synapsen).

*Gap-Junctions* besitzen Proteinporen aus Connexin-Proteinen mit einer Porengröße von etwa 1,5 Nanometer. Gap-Junctions stellen kein Membrankontinuum zwischen den Zellen her, sondern verbinden benachbarte Zellen über einen Abstand von 2 bis 4 Nanometer durch die Weiterleitung von elektrischen Signalen und den passiven Austausch kleiner Moleküle mit einem Molekulargewicht bis zu 1000 Dalton, in Ausnahmefällen bis zu 5000 Dalton. Der Signalfluss wird geregelt durch die Calcium-Konzentration und/oder das anliegende Potential, weshalb gap-Junctions auch als *"elektrische Synapsen"* bezeichnet werden. Ein Transport von Membranvesikeln ist nicht beschrieben. Gap-Junctions werden in Tier- und Pilzzellen gefunden. In Pflanzen wurden bislang nur Connexin-ähnliche Proteine identifiziert.

*Plasmodesmata* sind Membran-umgebene Cytoplasma-Kanäle. Sie verbinden benachbarte Pflanzenzellen über Poren in den Zellwänden mit einem Durchmesser von ca. 60 Nanometer. Dies führt zu einem Membrankontinuum zwischen den verbundenen Zellen. Auch die endoplasmatischen Retikuli von verbundenen Zellen können sich über Plasmodesmata erstrecken. Plasmodesmata sind für kleine Moleküle, Nährstoffe, Ribonucleoproteinkomplexe, Ionen und Fluoreszenzfarbstoffe durchlässig. Die Größenausschlussgrenze der Plasmodesmata liegt bei circa 1 bis 4 Kilodalton, aber auch einige sehr viel größere Viren verwenden Plasmodesmata als Infektionsweg. Ein Vesikeltransport über Plasmodesmata ist aber bislang nicht bekannt.

*Synaptische Verbindungen* sind Zellfortsätze (Axone), die an ihrem Ende über eine Synapse mit der Oberfläche einer anderen Zelle verbunden sind. Synapse und Zielzelle sind durch einen ca. 20 Nanometer breiten synaptischen Spalt getrennt. Der Durchmesser eines Axons beträgt ca. 0,3 bis 1,3 Mikrometer. Axone enthalten Mikrotubuli und ermöglichen einen bidirektionalen Vesikeltransport bis zur Synapse. Der Informationsaustausch erfolgt über die Ausschüttung von Signalstoffen an der Synapse. Diese Stoffe diffundieren über den synaptischen Spalt zur Zielzelle, wo sie durch Bindung an spezifische Rezeptoren zur Signalweiterleitung führen. Synaptische Verbindungen sind charakteristisch für das zentrale und periphere Nervensystem.

Die Kommunikation bzw. der Austausch von Information über längere Strecken erfolgt, soweit bekannt, über Cytoneme, Argosomen sowie über die Ausschüttung von Botenstoffen über das endokrine System.

*Cytoneme* sind feine circa 200 Nanometer dicke Zellausläufer, die zumeist frei im extrazellulären Raum enden, mitunter aber auch mit anderen Zellen in Kontakt stehen. Die Ausläufer enthalten Aktin, aber keine Mikrotubuli. Zur Zeit ist nicht bekannt, wie Cytoneme zur interzellulären Kommunikation beitragen. Cytoneme wurden nachgewiesen in Insekten (Drosophila, Imaginal-Disk-Zellen), Calpodes, Rhodnius und Seeigel-Embryos. Ein Vorkommen in Säugetierzellen ist bislang nicht beschrieben.

*Argosomen* sind intra- und extrazellulär vorkommende Membranvesikel, die Signalstoffe von Zelle zu Zelle transportieren können. Der Mechanismus des interzellulären Transports von Argosomen ist bisher nicht aufgeschlüsselt. Es wird postuliert, dass Argosomen über endo- und exozytotische Mechanismen transportiert werden. Argosomen wurden bislang nur in Drosophila-Embryos nachgwiesen.

Die *endokrine Kommunikation* zwischen Zellen zeichnet sich durch die Ausschüttung von Signalstoffen wie z. B. Hormonen in die Blutbahn aus, gefolgt von einer rezeptorvermittelten Wirkung auf die Zielzellen. Die endokrine Kommunikation ist ein generelles Prinzip in Organismen.

In einer Studie mit in vitro-Versuchen an Rattenzellen konnte kein Connexin bei der interzellulären Kommunikation nachgewiesen warden, wodurch die Gegenwart von gap junctions nicht gezeigt warden konnte. Allerdings werden von den Autoren dieser Studie die Anwesenheit von gap junctions weiter in Erwägung gezogen, und Membrantuben werden nicht erwähnt (Guérineau N. C. et al. Organotypic cultures of the rat anterior pituitary: morphology, physiology and cell-to-cell communication. Journal of Neuroscience Methods, 73, 169-176 (1997)).

Es ist Aufgabe der Erfindung, weitere Formen der Interaktion, des Transports und der Kommunikation zwischen Zellen aufzudecken. Es ist insbesondere Aufgabe der Erfindung, pharmazeutische und physikalische Mittel bereitzustellen, die die Kommunikation zwischen den Zellen und den interzellulären Transport beeinflussen können. Ferner ist es Aufgabe der Erfindung, Mittel und Verfahren zur Untersuchung der neuen Formen der Zellkommunikation und des interzellulären Transports zur Verfügung zu stellen.

### Zusammenfassung der Erfindung

Diese Aufgabe wird gelöst durch ein Verfahren zur Untersuchung von interzellulärer Kommunikation und interzellulärem Transport, indem Zellen nach Vereinzelung auf Membrantuben hin untersucht werden, die F-Actin und Myosin enthal-ten, die einen Durchmesser von 50 bis 400 Nanometer besitzen, die bis zu 50 Mikrometer lang sind und die sich zwischen den Zellen spannen.

Ein zweiter Aspekt der Erfindung betrifft die Untersuchung des Organellentransports zwischen den Zellen über derartige Membrantuben, beispielsweise durch die die Schritte: (i) Zusetzen von ein oder mehreren Stoffen zu einer ersten Anzahl Zellen, wobei der Stoff so gewählt ist, dass er innerhalb einer ersten Zeitdauer von den Zellen der ersten Anzahl endozytiert wird; (ii) Mischen der ersten Anzahl Zellen nach Waschen mit einer zweiten Anzahl Zellen, so dass sich zwischen den Zellen der ersten und der zweiten Anzahl innerhalb einer zweiten Zeitdauer interzelluläre Membrantuben bilden, und (iii) Bestimmen der Zahl der Zellen aus der zweiten Anzahl Zellen, die die ein oder mehrere endozytierte Substanzen enthalten. In einer anderen Ausführungsform wird in Schritt (i) die erste Anzahl Zellen mit einem ersten endozytierbaren Stoff und die zweite Anzahl Zellen mit einem zweiten endozytierbaren Stoff versetzt wird, wobei erste und zweite Substanz verschieden sind. Die endozytierbaren Substanzen können gewählt, werden aus ein oder mehreren Farbstoffen, Fluoreszenzfarbstoffen, Dil, DiO, Lysotracker™, radioaktiven Markersubstanzen, Lumineszenzfarbstoffen, fluoreszierenden oder lumineszierenden Proteinen und Peptiden, Proteinen oder Peptiden, die gekoppelt sind mit einer Markersubstanz gekoppelt sind. Diese Untersuchung können alternativ ach so erfolgen, dass in Schritt (i) nich eine endozytierte Substanz verfolgt wird, sondern dass man zuerst eine konstitutive oder transiente Expression einer Nachweissubstanz in die Organellen erzielt. Der Schritt (iii) kann durch FACS erfolgen.

In einem dritten Aspekt der Erfindung erfolgt die Untersuchung des Organellentransports in Anwesenheit bzw. unter Einwirkung eines Prüfmittels erfolgt. Das Prüfmittel kann eine chemische Verbindung oder eine mutmaßliche pharmazeutische Wirksubstanz sein, bevorzugt ein Arzneimittel oder Therapeutikum. Das Prüfmittel kann auch eine physikalische Vorrichtung sein, bevorzugt eine physikalisch therapeutische Vorrichtung.

In einem weiteren Anspekt der Erfindung erfolgt die Untersuchung mit einem mikroskopischen System, das die Beobachtung von verschiedenen mikroskopischen Ebenen in der Z-Achse erlaubt. Das mikroskopische System umfasst bevorzugt ein Mikroskop, einen z-Stepper und eine zugehörige Steuerung.

Ein weiterer Aspekt der Erfindung betrifft die Verwendung des genannten Verfahrens und der Vorrichtungen zur Reihenuntersuchung von mutmaßlichen Arzneimitteln und Wirkmitteln., insbesondere zur Reihenuntersuchung von mutmaßlichen Wirkstoffen und Wirkmitteln zur Behandlung von Tumoren, des Bluthochdruck, von viralen, bakteriellen oder parasitischen Infektionserkrankungen, Erkrankungen des Stoffwechsels, Erkrankungen des Nervensystems, der Psyche und des Geistes, und des Cholesterinspiegels. Eine weitere Verwendung des erfindungsgemäßen Verfahrens liegt in der Untersuchung von Wirkstoffen in der Gentherapie, zum Zelltargeting und in der Pharmakologie. Die Erfindung betrifft auch pharmazeutische Zusammensetzungen, die so ermittelte Wirkstoffe enthalten und auch therapeutische Verfahren auf Grundlage der Wirkmittel zur Beeinflussung der interzellulären Kommunikation und des interzellulären Organellentransports.

Weitere Vorteile, Aufgaben und Merkmale der Erfindung ergeben sich aus den Beispielen und den anliegenden Figuren.

### Beschreibung der Abbildungen

Es zeigt:

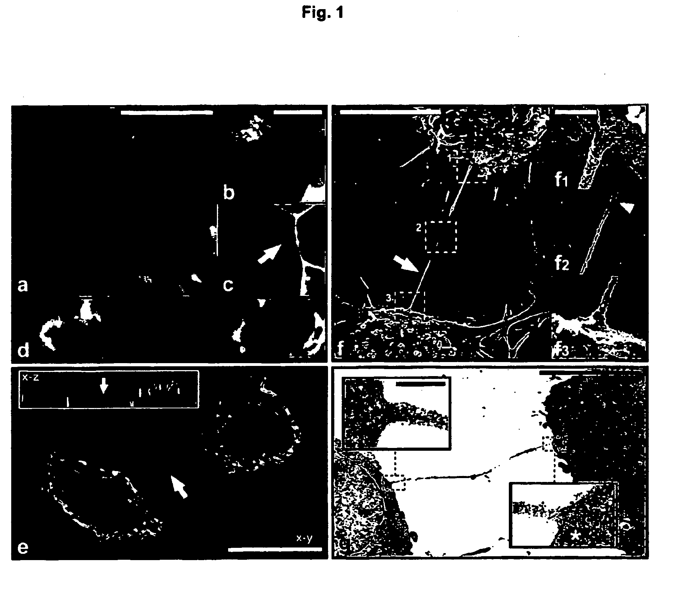

- Fig. 1a: hochauflösende, dreimensionale Videomikroskopieaufnahme von lebenden PC12-Zellen circa 24 Stunden nach Zellpassage und Färbung mit WGA und einem TNT (Bildmitte), das zwei PC12-Zellen über einen sehr weiten Abstand verbrückt (Balken:15 Mikrometer);
- Fig. 1b: Aufnahme gemäß Fig. 1a, die mehrere TNTs zeigt, die sich von einer Zelle ausgehend zu verschiedenen Zellen erstrecken (Balken: 15 Mikrometer);
- Fig. 1c: Aufnahme gemäß Fig. 1a von einem verzweigten TNT (siehe Pfeil);
- Fig. 1d: ein rechnerischer (x-z)-Einzelschnitt eines TNTs, das ohne Berührung des Substrats zwischen zwei PC12-Zellen aufgespannt ist;
- Fig. 1e: ein (x-y)-Einzelschnitt aus der dreidimensionalen Rekonstruktion einer videomikroskopischen Mehrfarben-Fluoreszenzaufnahme von fixierten, über TNTs verbundenen PC12-Zellen nach Immunfärbung mit einem Anti-Tubulin-Antikörper (grün), FITC-Phalloidin-Aktinfärbung (rot) und Dapi-Kemfärbung (blau, Molecular Probes D-1306) - das Vorkommen von Tubulin ist auf die Zellkörper beschränkt, während das Vorkommen von Aktin sich über die TNTs erstreckt (Pfeil); der Einsatz zeigt den entsprechenden (x-z)-Einzelschnitt durch das pfeilmarkierte TNT (Balken: 15 Mikrometer);
- Fig.1f: eine Raster-Elektronenmikroskopieaufnahme von TNTs: PC12-Zellen wurden 30 Minuten mit 2,5% Glutaraldehyd in 0,1 M Phosphatpuffer (pH 7,4), ergänzt mit 1% Saccharose fixiert und in üblicher Weise für die Mikroskopie vorbereitet: f) TNT zwischen zwei Zellen; f1) f2) und f3) sind vergrößert Ausschnitte der eingekästelten Bereiche aus (f) und zeigen die Basisteile und den Mittelbereich des TNTs mit einem Durchmesser von ca. 50 Nanometer - die Basisteile bilden offenbar ein Membrankontinuum mit den Plasmamembranen der verbundenen Zellen, (Balken: großes Bild 15 Mikrometer, kleine Bilder 200 Nanometer);
- Fig. 1g: Transmissions-Elektronenmikroskopische Aufnahme eines TNTs mit einem Durchmesser von 50 nm, das zwei fixierte PC12-Zellen verbindet. Die Boxen zeigen Vergrößerungen der TNT-Basen. Stern, sekretorisches Granulum, (Balken: großes Bild 10 Mikrometer, kleine Bilder 200 Nanometer);

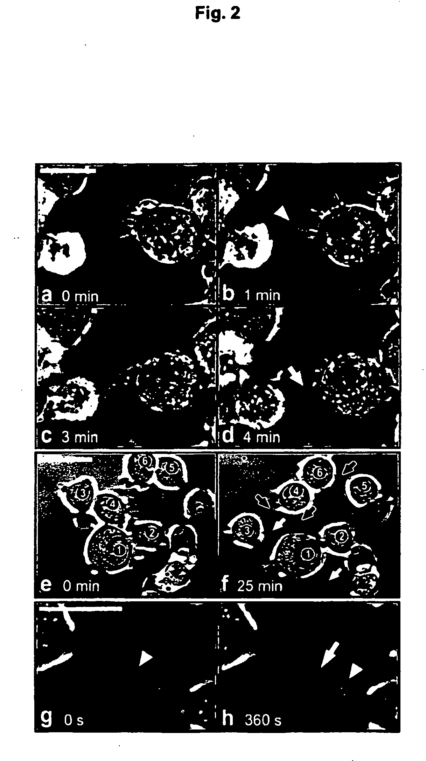

- Fig. 2a-d: zeitversetzte Hellfeld-Videomikroskopieaufnahmen von *de novo* gebildeten TNTs in verschiedenen Stadien 2 Stunden nach Ausplattierung von PC12-Zellen: a) dynamische Vorsprünge an einer Zelle; b) der durch eine Pfeilspitze markierte Vorsprung trifft in c) auf eine benachbarte Zelle und bildet ein TNT (Pfeil) aus; angegebene Zeiten in Minuten (min), (Balken: 20 Mikrometer);
- Fig. 2e, f: zeitversetzte Hellfeld-Videomikroskopieaufnahmen von der Bildung eines TNT-Netzwerks in einer PC12-Zellkultur: es werden einerseits - vermutlich bereits bestehende- TNTs (schwarze Pfeile) zwischen auseinander laufenden Zellen sichtbar und es werden neue TNTs (weiße Pfeile) gebildet, so dass ein komplexes Netzwerk entsteht (Abbildungen sind Überlagerungen von drei (x-y)-Einzelschnittebenen und zeigen alle erfassbaren TNTs; gleiche Zellen sind gleich nummeriert, Zeitangaben in Minuten (min), (Balken: 20 Mikrometer);
- Fig. 2g, h: erstes und letztes Einzelbild einer direkten Hellfeld-Videomikroskopiesequenz, die über einen Zeitraum von 360 Sekunden vom unidirektionalen, interzellulären Organellentransport (Pfeilspitze) in dem zwischen zwei PC12-Zellen aufgespannten TNTs, circa 50 Minuten nach Zellpassagedie Transportgeschwindigkeiten lagen zwischen 8 und 100 Nanometer pro Sekunde. Der Pfeil markiert den Ausgangspunkt der Translokation, (Balken: 20 Mikrometer);

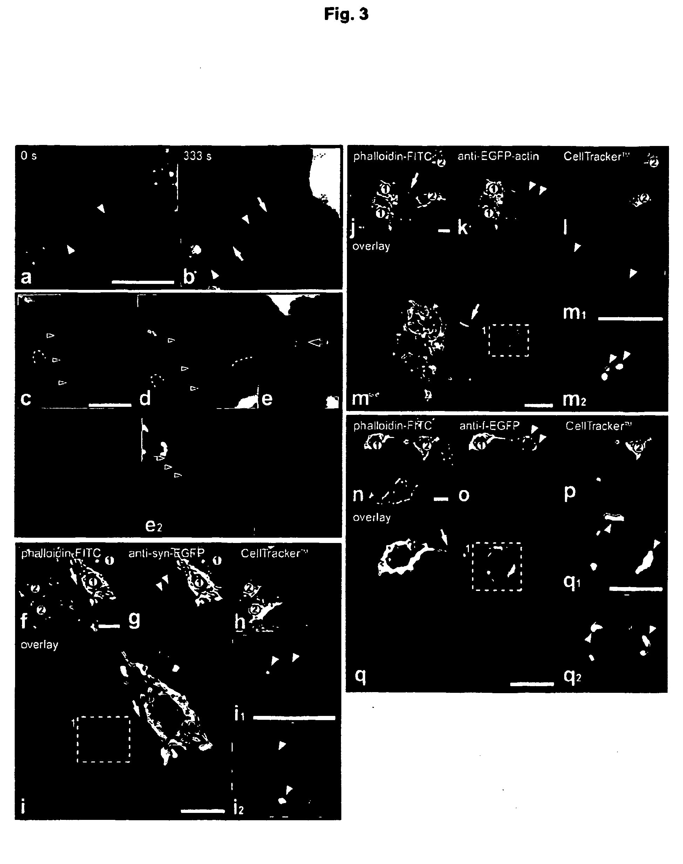

- Fig. 3a, b: Fluoreszenz-Videomikroskopieaufnahmen von PC12-Zellen nach Färbung mit Lysotracker^{™} - die fluoreszierenden Organellen (Pfeilspitzen) werden unidirektional im TNT bewegt, wobei Pfeile die Ausgangspunkte der Objekte markieren, (Balken: 10 Mikrometer);
- Fig. 3c-e2: Fluoreszenz-Videomikroskopieaufnahmen von PC12-Zellen nach Antikörper-Färbung von Synaptophysin (grün in c, e), Myosin Va (grün in d und rot in e) und Rab 3a (grün in e2) sowie TRITC-Phalloidin-Aktinfärbung (rot in c, d, e2 und blau in e) - offene Pfeilspitzen markieren Punktsignale und gestrichelte Linien Teile der Zellränder; Synaptophysin- und Myosin-Va-Signale waren an selber Stelle zu finden (offener Pfeil in e); die jeweils punktförmigen Färbungen legen nahe, dass in TNTs Synaptophysin-positive Organellen (z. B. SLMVs) von Motorproteinen wie z. B. Myosin Va fortbewegt werden und regulatorische Proteine wie z. B. Rab3a an diesem Transport beteiligt sind, (Balken: 10 Mikrometer);
- Fig. 3f-i2: TNT-vermittelter Transfer von Synaptophysin-EGFP. Eine Population von PC12-Zellen wurde mit CellTracker^{™} (Molecular Probes, "blue" C-2110) gefärbt (Population 2) und mit einer weiteren Population PC12-Zellen, die mit Synaptophysin-EGFP (syn-EGFP) transfiziert wurde (Population 1) gemischt ausplattiert. Die gemischten Zellkulturen wurden 24-48 Stunden später fixiert und immuncytochemisch mit einem polyklonalen anti-GFP Antikörper (Molecular Probes A-6455) und Phalloidin-FITC gefärbt. Die Zellen wurden mittels dreidimensionaler-Fluoreszenzmikroskopie und anschließender Dekonvolution der Bilddaten analysiert. In den Einzelkanal-Aufnahmen sind korrespondierende (x-y)-Einzelschnitte durch mit TNTs (Pfeile) verbundenen Zellen beider Population dargestellt (obere Bildreihe, Nummern markieren die korrespondierenden Zellpopulationen). Man beachte, dass Synaptophysin-EGFP in Form punktierter Signale selektiv in TNT-verbundenen Zellen von Population 2 nachgewiesen wurde (Pfeilspitzen), d. h. selektiv zwischen diesen Zellen transferiert wurde. In der Überlagerung (overlay) der Einzelkanäle ist das Phalloidin-Signal grün, das Synaptophysin-Signal rot und das CellTracker™-Signal blau dargestellt. Für die Einkästelung ist eine Vergrößerung der korrespondieren (i1) als auch einer zusätzlichen (x-y)-Einzelschnittebene (i2) dargestellt, (Balken: 10 Mikrometer);
- Fig. 3j-m2: TNT-vermittelter Transfer von EGFP-Aktin. Abbildung gemäß Abbildung 3f-i2, jedoch wurden die Zellen von Population 1 mit EGFP-Aktin (EGFP-actin, Fischer, M., Kaech, S., Knutti, D. & Matus, A. Rapid Actin-Based Plasticity in Dendritic Spines. Neuron (1998) 20: 847-854) transfiziert. Man beachte, dass EGFP-Aktin selektiv in über TNT verbundenen Zellen von Population 2 nachgewiesen wurde (Pfeilspitzen), d. h. selektiv zwischen den TNT-verbundenen Zellen transferiert wurde;
- Fig. 3n-q2: TNT-vermittelter Transfer von famesyliertem EGFP (f-EGFP). Abbildung gemäß Abbildung 3f-i2, jedoch wurden die Zellen von Population 1 mit f-EGFP (pEGFP-f, Clontech 6074-1) transfiziert. Man beachte, dass f-EGFP selektiv an der Plasmamembran von mit TNTs verbundenen Zellen von Population 2 nachgewiesen wurde (Pfeilspitzen), d. h. in Form eines Plasmamembrantransfers selektiv zwischen den TNT-verbundenen Zellen ausgetauscht wurde;

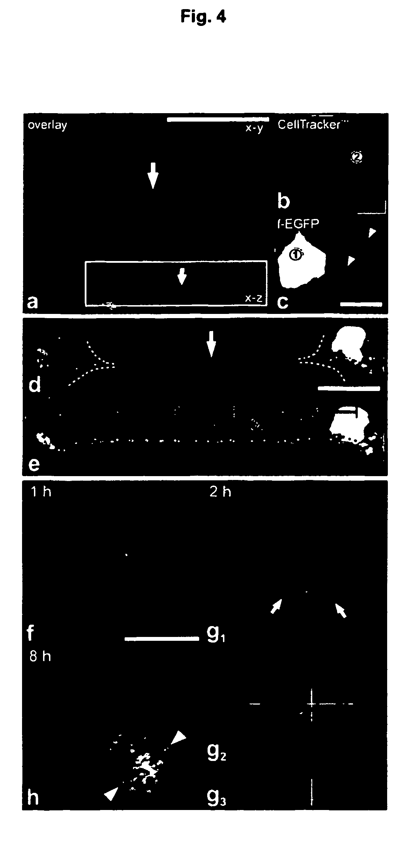

- Fig. 4a-c: TNT-vermittelter Transfer von famesyliertem EGFP (f-EGFP) beobachtet an lebenden Zellen. Eine Population von PC12-Zellen wurde mit Cell-Tracker^{™} (Molecular Probes, "blue") gefärbt (Population 2) und mit einer weiteren Population PC12-Zellen, die mit f-EGFP (Clontech) transfiziert wurde (Population 1) gemischt ausplattiert. Die gemischten Zellkulturen wurden 48 Stunden später mittels dreidimensionaler-Fluoreszenz-Video-mikroskopie analysiert. In den Einzelkanal-Aufnahmen (b, c) sind korrespondierende (x-y)-Einzelschnitte duch mit TNTs verbundenen Zellen beider Population dargestellt (Nummern markieren die korrespondierenden Zellpopulationen). In der Überlagerung (overlay) der Einzelkanäle ist das f-EGFP-Signal grün und das CellTracker™-Signal blau dargestellt. Die Einschubbox in der Überlagerung zeigt einen korrespondierenden (x-z)-Einzelschnitt duch des markierte TNT (Pfeil). Man beachte, dass f-EGFP als Plasmamembransignal in Zellen von Population 2 nachgewiesen wurde (Pfeilspitzen), d. h in Form eines Plasmamembrantransfers selektiv zwischen den TNT-verbundenen Zellen ausgetauscht wurde, (Balken: 20 Mikrometer);

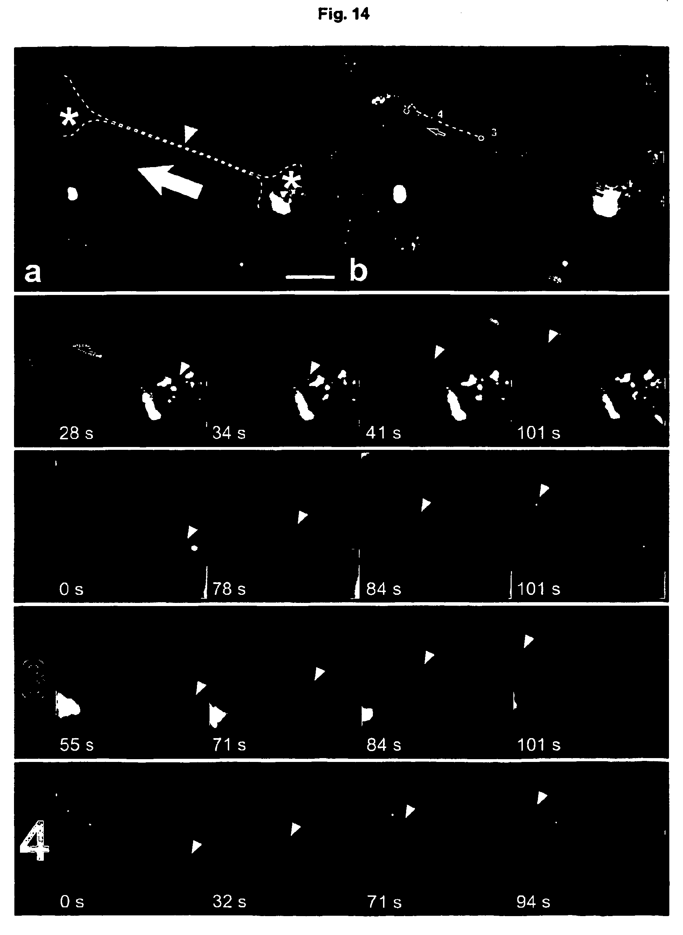

- Fig. 4d-e: Direkter Transfer von Dil positiven Organellen zwischen TNT verbundenen Zellen. PC12-Zellen wurden mit Dil gefärbt und auf LabTek^{™} Kulturschalen ausplattiert (siehe Beispiel 14). Die Zellen wurden anschließend mittels Fluoreszenzvideomikroskopie analysiert. Gezeigt sind Einzelbilder dreier Videosequenzen, die den unidirektionalen Transfer mehrer Dil positiver Organellen zwischen zwei über ein TNT (Pfeil) miteinander verbunden Zellen zeigen. Von vier representativen Organellen sind ihre teilweise überlappenden Trajektorien dargestellt (Pfeile 1-4). Gestrichelte Linien markieren Teile der Zellumrisse (für Details siehe Abbildung 14), (Balken: 20 Mikrometer);
- Fig. 4f-h: konfokale, dreidimensionale Fluoreszenzmikroskopieaufnahmen des Transports zwischen zwei gemischten PC12-Zell-Populationen, jede für sich gefärbt mit Dil (rot) und DiO (grün), zu verschieden Zeitpunkten nach der Ausplattierung vereinzelter Zellen: f) eine Stunde alte Zellen enthalten entsprechend entweder nur Dil- oder nur DiO-positive Vesikel; g1) nach zwei Stunden enthalten einzelne Zellen sowohl Dil- als auch DiO-positive Strukturen (Pfeile); g2 ist eine dreidimensionale Analyse des zugehörigen (x-y)- bzw. (x-z)-Einzelschnitts (g3) der Zelle aus g1 und zeigt, dass Dilpositive Organellen sich innerhalb einer DiO-markierten Zelle befinden; h) 8 Stunden später enthalten einzelne Zellen weitgehend Vesikel (Pfeilspitzen), die für beide Farbstoffe positiv (gelb) sind, (Balken: 20 Mikrometer);

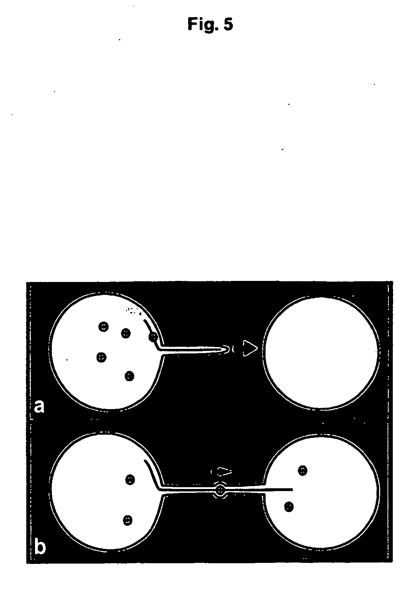

- Fig. 5: zeichnerische Darstellung des TNT-Konzepts mit Deutung der erzielten Ergebnisse. Eine Zelle bildet aktin-abhängig in Richtung einer Zielzelle einen Fortsatz aus (a). Nach Fusion des Fortsatzes mit der Membran der Zielzelle und Ausbildung eines Membrankontinuums zwischen beiden Ziellen (b), d. h. Ausbildung eines TNTs, können Organellen unidirektional zwischen Zellen transferiert werden. Rot, Aktin; grün, Organellen;

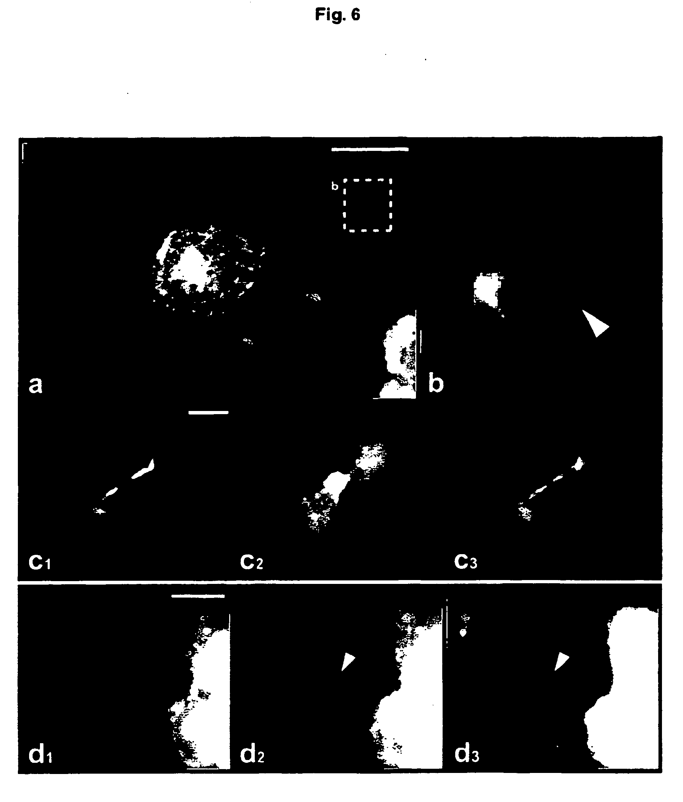

- Fig.6: TNTs enthalten filamentöses Aktin aber keine Mikrotubuli. PC12-Zellen wurden 24 Stunden nach dem Ausplattieren mit Glutaraldehyd fixiert (für Details siehe Fig. 1f), immuncytochemisch mit Antikörpern gegen Alpha-Tubulin (a, b, c1, d1) und mit Phalloidin-FITC (c2, d2) gefärbt und anschießend mittels Fluoreszenzmikroskopie analysiert. Man beachte die feinen Mikrotubulistränge in den Filopodien der Zellen (a). Ein solcher Bereich (Einkästelung in a) ist in (b) vergrößert dargestellt und die Pfeilspitze markiert einen Mikrotubulistrang. In Zellen, die während der Zytokinese fixiert wurden, sind die gebündelten Mikrotubuli der "Midbody"-Region (c1) und der kontraktile Aktinring zwischen den Zellen (c2) zu beobachten. (c3) zeigt eine Überlagerung der Bilder von (c1) und (c2). Bei TNT-verbundenen Zellen (d1-d3) zeigt sich, dass in dem TNT (Pfeilspitze) keine Mikrotubuli (d1), dagegen jedoch filamentöses Aktin (d2) nachgewiesen werden kann (vergleiche auch Fig. 1e). (d3) zeigt eine Überlagerung der Bilder von (d1) und (d2), (Balken: 10 Mikrometer);

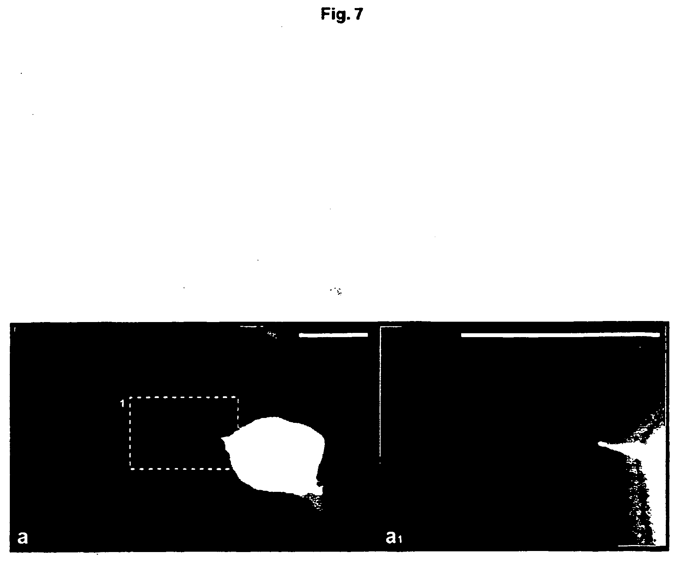

- Fig. 7: Zytoplasmatisches EYFP wird nicht zwischen TNT-verbundenen Zellen transferiert. Abbildung gemäß der Überlagerung (overlay) von Abbildung 3f-i2, jedoch wurden die Zellen von Population 1 mit EYFP (Clontech) transfiziert und die mikroskopischen Bilddaten nicht dekonvolviert. Das Signal vom Phalloidin gefärbten, filamentösen Aktin ist grün, das EYFP-Signal rot und das CellTracker™-Signal blau dargestellt. Für die Box in (a) ist eine Vergrößerung (a1) dargestellt. Man beachte, dass EYFP nicht in den TNT verbundenen Zellen der Population 2 (blaue Zellen) nachzuweisen ist und die aufgespannten TNTs nur bis ca zur Hälfte eindringt, d. h. nicht effizient durch TNTs transferiert wird, (Balken: 10 Mikrometer);

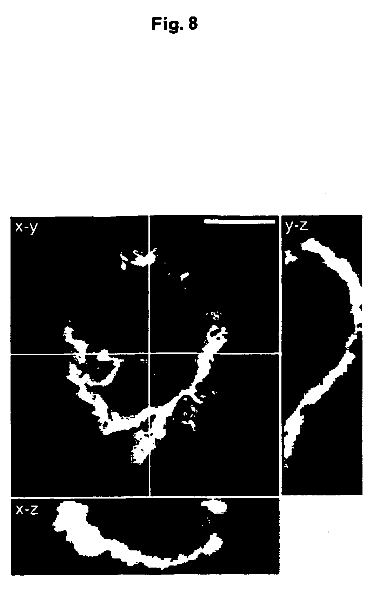

- Fig. 8: Famesyliertes EGFP (f-EGFP) ist ein sehr spezifischer Plasmamembranmarker. PC12-Zellen wurden gemäß Fig. 3n-q2 zur mikroskopischen Analyse vorbereitet. f-EGFP exprimierende Zellen wurden anschießend mittels dreidimensionaler konfokaler Fluoreszenzmikroskopie analysiert. Gezeigt ist ein (x-y)-Einzelschnitt als auch korrespondierende (x-z)- bzw. (y-z)-Einzelschnitte (die jeweiligen Schnittebenen sind als Linien im (x-y)-Einzelschnitt dargestellt) durch die Mitte einer Zelle. Man beachte, dass f-EGFP ausschließlich an der Zellplasmamembran lokalisiert ist, (Balken: 10 Mikrometer);

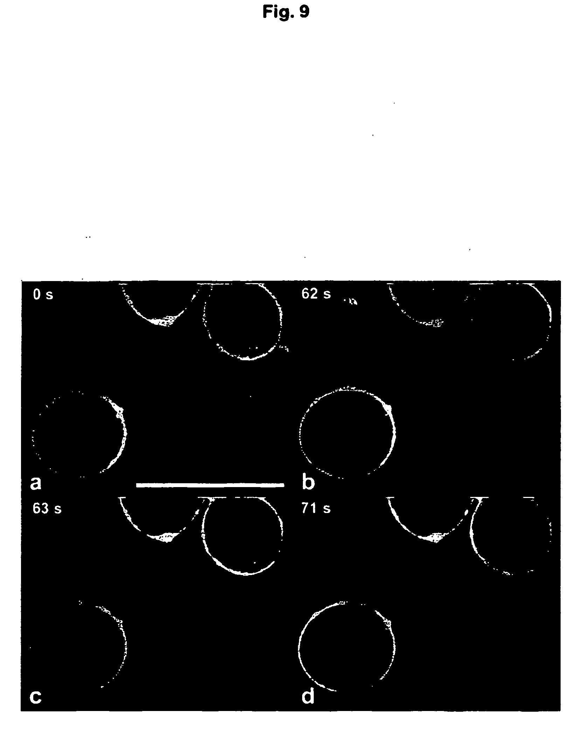

- Fig. 9a-d: vier Videomikroskopieaufnahmen gemäß Fig. 1a von einem TNT über einen Beobachtungszeitraum von ca. 70 Sekunden, in dem die Verbindung (a) von dem einfallenden Licht der Wellenlänge 565 nm zunächst in Schwingung versetzt wird (b), reißt (c) und sich innerhalb Sekunden an seinem losen Ende wie ein gerissenes Gummiband aufwickelt (d). Die Zeitpunkte der Bildaufnahmen sind in Sekunden (s) angegeben, (Balken: 20 Mikrometer);

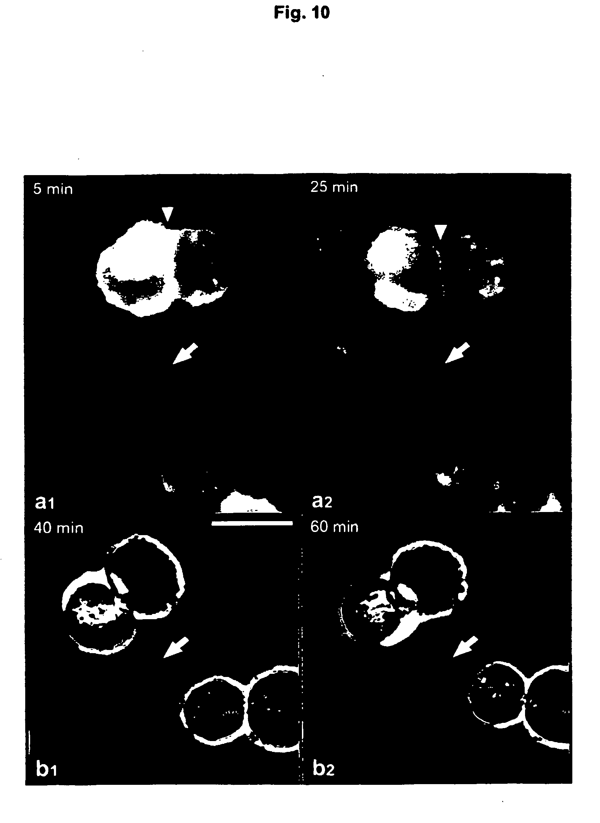

- Fig. 10: zeitversetzte Fluoreszenz- und Hellfeld-Videomikroskopieaufnahmen von TNTs zwischen PC12-Zellen, die in Zellmedium einer Behandlung mit 1,25% (a1, a2) bzw. 2,5% (b1, b2) Trypsin/EDTA unterzogen wurden: die andauernde Trypsin/EDTA-Behandlung führt zur Ablösung der Zellen vom Substrat (sichtbar durch die zunehmende Aufrundung der Zellen (Pfeilspitzen)) und schließlich zu deren Ablösung; die TNTs bleiben über den beobachteten Zeitraum intakt (Pfeile). Die Zeitpunkte der Bildaufnahmen sind in Minuten (min) angegeben, (Balken: 20 Mikrometer);

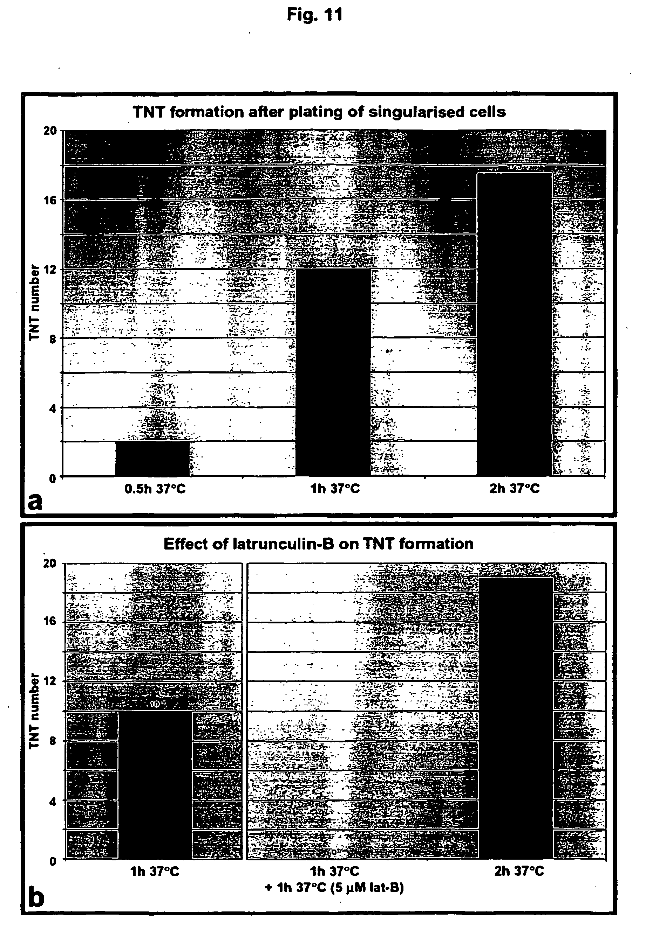

- Fig. 11 a: eine graphische Darstellung des zeitlichen Auftretens von TNTs bei PC12-Zellen in Abwesenheit von Latrunculin B. Abszisse: gemessene Zeitpunkte nach Zellpassage in Stunden (h); Ordinate. Anzahl der in 10 zufällig ausgewählten Bereichen des Zellkulturgefäßes *de novo* ausgebildeten TNTs, bestimmt durch eine Plasmamembranfärbung der Zellen mit WGA und dreidimensionaler fluoreszenzmikroskopischer Analyse.
- Fig. 11b: eine graphische Darstellung des zeitlichen Auftretens von TNTs bei PC12-Zellen in Gegenwart von 5 Mikromolar Latrunculin B. Abszisse: gemessene Zeitpunkte nach Zellpassage in Stunden (h) in Gegenwart (+) und Abwesenheit (-) von Latrunculin B nach Zellpassage; Ordinate: Anzahl der in 10 zufällig ausgewählten Bereichen des Zellkulturgefäßes *de novo* ausge-bildeten TNTs, bestimmt durch eine Plasmamembranfärbung der Zellen mit WGA und dreidimensionaler fluoreszenzmikroskopischen Analyse.

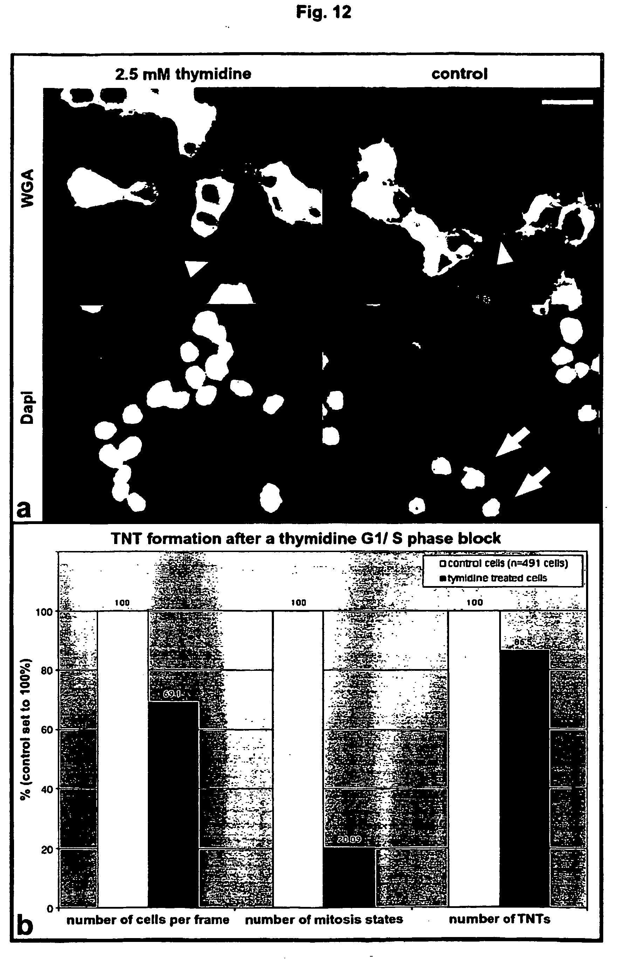

- Fig. 12a: Fluoreszenzmikroskopische Aufnahme von PC12-Zellen circa 1 Stunde nach Zellpassage und Färbung mit WGA, 20 Minuten Fixierung in 4% Paraformaldehydl 4% Sucrose, 3 Minuten Behandlung mit 0,2% Triton™ X-100 und Kemfärbung mit Dapi - linke Abbildungen in (a): die 14-stündige Behandlung der Zellen vor der Passage mit 2,5 mM Thymidin im Zellkulturmedium blockierte die Zellen in der G1/S-Phase des Zellzyklus und verhinderte eine Teilung der Zellen; rechte Abbildungen in (a): in unbehandelten PC12-Zellen (Kontrolle) sind in der Kernfärbung Mitosestadien (Pfeile) zu beobachten. Trotz der Abwesenheit von Mitosestadien bilden Thymidin-behandelte PC12-Zellen TNTs aus (Pfeilspitzen), (Balken: 20 Mikrometer);
- Fig. 12b: eine graphische Darstellung und quantitative Analyse der Ergebnisse von Fig. 12a - die Anzahl der Mitosestadien wurde gegenüber der Kontrolle durch Thymidinbehandlung auf 20% reduziert, aber die TNT-Bildung blieb mit 86,5% erhalten (die geringe Reduktion beruht vermutlich auf der geringeren Zahl der Zellen bedingt durch den Zellteilungsblock); die TNTs sind somit kein Zellteilungsphänomen oder das Ergebnis einer unvollständig abgelaufenen Zytokinese;

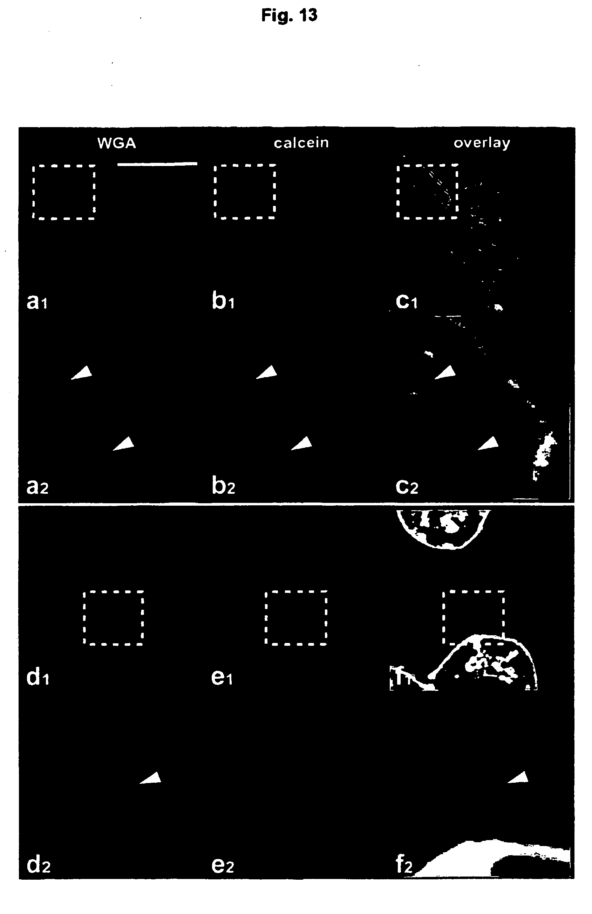

- Fig. 13:: Calcein dringt effizient in Filopodien aber nicht in TNTs ein. PC12-Zellen wurden 24 Stunden nach dem Ausplattieren mit WGA (linke Säule, rot) und Calcein AM (mittlere Säule, grün, Molecular Probes C-3099, 1:3000) gefärbt und anschließend mittels konfokaler Fluoreszenzmikroskopie analysiert (die rechte Säule zeigt die Überlagerung (overlay)). Die Analyse von Filopodien in optischen (x-y)-Einzelschnitte am Zellboden (a1-c2), wo diese Strukturen zahlreich vorkommen, zeigt, dass Calcein auch dünne Filopodien effizient färbt. Für die eingekästelten Bereiche in a1, b1, c1 sind jeweils Vergrößerungen dargestellt (a2, b2, c2), in denen Pfeilspitzen die mit Calcein gefärbten Filopodien markieren. Bei der Analyse von TNTs (d1-f2) zeigen optische (x-y)-Einzelschnitte durch die Zellmitte, wo diese Strukturen typischerweise lokalisiert sind, dass Calcein nicht in diese eindringt. Für die eingekästelten Bereiche in d1, e1, f1 sind jeweils Vergrößerungen dargestellt (d2, e2, f2), in denen Pfeilspitzen das zwischen den Zellen aufgespannte TNT markieren, (Balken: 15 Mikrometer);
- Fig. 14:: Direkter Transfer von Dil-positiven Organellen zwischen TNT-verbundenen Zellen. Abbildung gemäß Abbildung 4d-e. In dem Übersichtsbild (a) sind zwei, über ein TNT (Pfeilspitze) verbundene Zellen (Sterne) gezeigt, das den unidirektionalen Transfer (in Pfeilrichtung) von Dil positiven Organellen zeigt. Die gestrichelten Linien markieren Teile der Zellumrisse. In (b) sind die teilweise überlappenden Trajektorien von vier repräsentativen Organellen, als farbkodierte und numerierte gestrichelte Linien dargestellt (1-4). Die farbigen Pfeile markieren die jeweilige Richtung des Transfers. Für jede in (b) gezeigte Trajektorie sind vier ausgewählte Einzelbilder der zugrundeliegenden Videosequenz dargestellt (Reihen 1-4), die den Transport der korrespondierenden Organellen (Pfeilspitzen) zeigen. Die Zeitpunkte der Bildaufnahme sind in Sekunden (s) angegeben, (Balken: 20 Mikrometer);

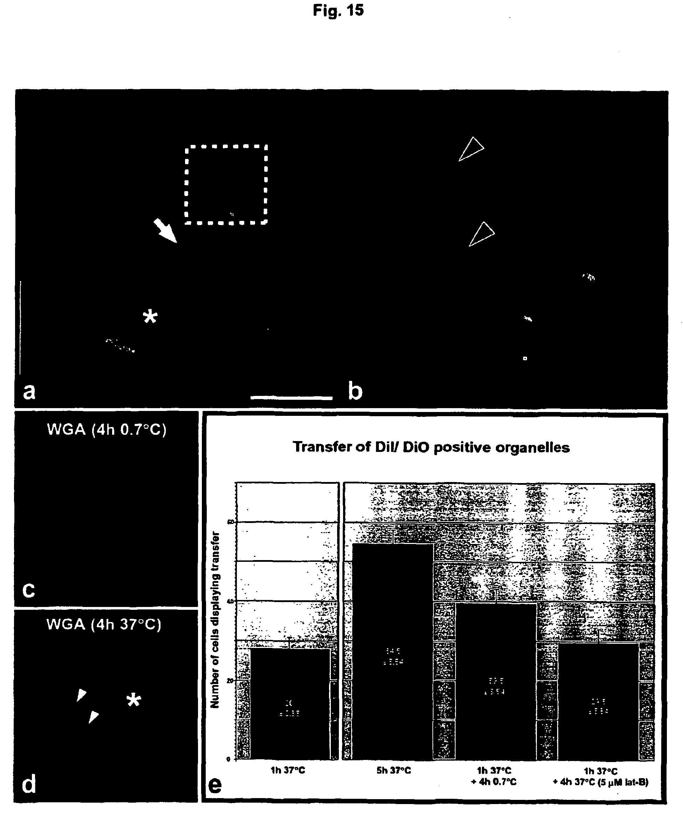

- Fig. 15: einen fluoreszenzmikroskopischen Nachweis unter Anwendung eines Temperaturblocks, dass TNTs den interzellulären Transfer von Dil/ DiO gefärbten Organellen nach einem Mechanismus bewerkstelligen, der gängige endo- und exozytotische Mechanismen ausschließt. (e) Zwei unterschiedlich gefärbte PC12-Zellpopulationen werden als Mischkultur auf LabTek™-Kulturschaten für 1 Stunde bei 37° C kultiviert und dann bei verschiedenen Temperaturen in Abwesenheit oder in Gegenwart von 5 Mikromolar Latrunculin-B (lat-B) weiter kultiviert. (a) Zwei unterschiedlich gefärbte PC12-Zellpopulationen werden als Mischkultur auf LabTek™-Kulturschalen für 1 Stunde bei 37° C kultiviert und dann für 4 Stunden bei 0.7° C. Zu sehen ist eine vereinzelte, doppelt gefärbte Zelle, die mit einem TNT (Pfeil) zur Nachbarzelle (Stern) verbunden ist. (b) Vergrößerung der eingekästelten Zellregion in (a). (c, d) PC12-Zellen wurden 1 Stunde nach Ausplattierung mit WGA gefärbt, anschließend für weitere 4 Stunden bei 0.7° C (c) oder 37° C (d) kultiviert und dann videomikroskopisch analysiert. Zellen in (c) zeigen ausschließich eine Plasmamembran-Färbung, während Zellen in (d) zusätzlich eine perinukleäre Färbung (Stern) als auch gefärbte endozytotische Strukturen (Pfeilspitzen) zeigen, (Balken: 10 Mikrometer);

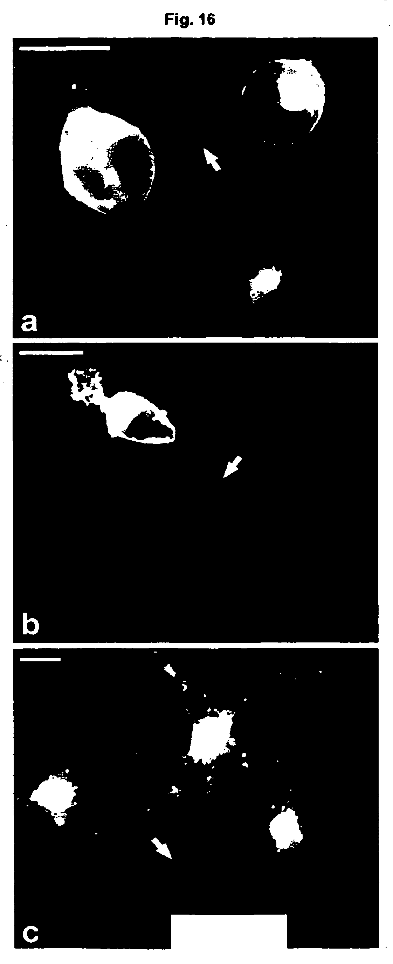

- Fig. 16: eine (x-y)-Einzelschnittebene einer dreidimensionalen fluroeszenz-video-mikroskopischer Aufnahme. Gezeigt sind nicht-neuroendokrinen HEK-293-Zellen (a, ATCC CRL 1573 - menschliche embryonale Nierenzellen), Medulla-Primärkulturen der Ratte (b, zur Isolierung vereinzelter Zellen wurde das Nebennierenmark von P10-Ratten einer Collagenase- (0.1%) und wiederholten Trypsin-Behandlungen (0.125%) unterzogen; die vereinzelten Zellen wurden anschließend auf Polyomithin/Laminin beschichtete LabTek™ Zellkulturschalen ausplattiert und 4 Tage kultiviert) sowie hippocampale Primärkulturen (c, präpariert nach Standardmethoden (Banker, G. Goslin, K. in Culturing Nerve Cells, eds. Banker G & Goslin K, MIT Press, Cambridge MA, 1991)) nach Färbung mit WGA. Die in den Kulturen gefundenen TNTs (Pfeile) berühren nicht das Substrat und überspannen die kürzeste Strecke zwischen den jeweiligen Zellen (Balken: 20 Mikrometer) ;

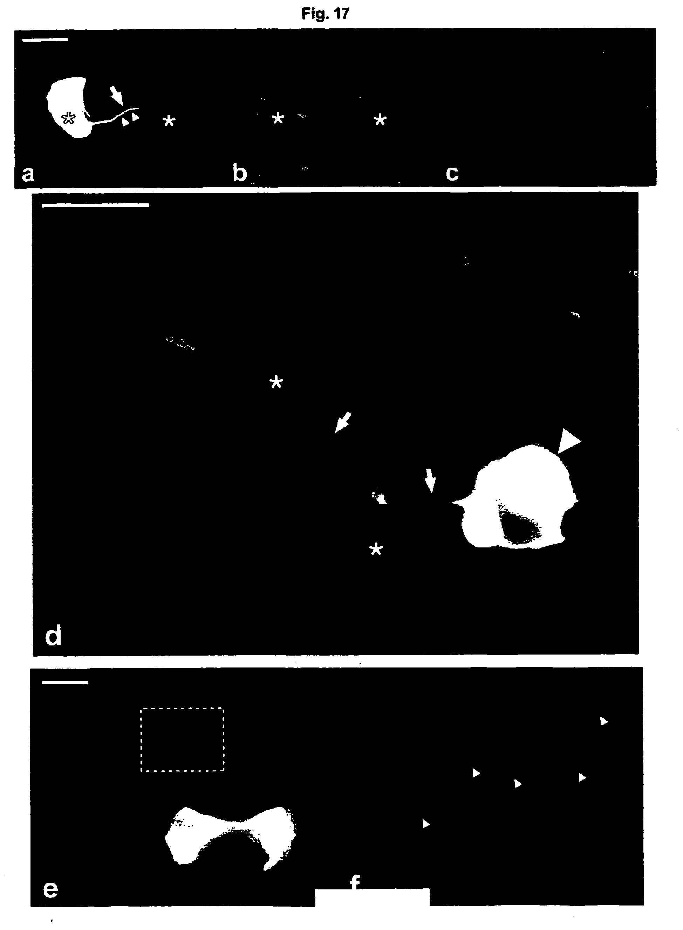

- Fig. 17a: eine fluoreszenzmikroskopische Aufnahme von PC12-Zellen, die ca. 24 Stunden nach Transfektion mit VP22-GFP analysiert wurden. Auffällig ist eine stark fluoreszierende TNT-Verbindung (Pfeil) zwischen einer stark und einer schwach positiven Zelle (Sterne). Innerhalb dieser Verbindung zeigen sich stark fluoreszierende, vesikuläre Strukturen (Pfeilspitze), die im Verlauf der Beobachtung ihre Position veränderten.
- Fig. 17b: Zu Abb 17a korrespondierende transmissionsmikroskopische Aufnahme. Die Sterne markieren dieselben Zellen wie in Fig. 17a.
- Fig. 17c: eine Überlagerung der Fig. 17a (rot) und 17b (blau).
- Fig. 17d: eine fluoreszenzmikroskopische Analyse von PC12-Zellen 48 Stunden nach der Transfektion mit VSVG-ECFP (Rustom, A., Bajohrs, M., Kaether, C., Keller, P., Toomre, D., Corbeil, D., Gerdes, H.-H.: Selective delivery of secretory cargo in Golgi-derived carriers of non-epithelial cells, in Traffic 2002, 3: 279-288) und Ausplattieren auf LabTek^{™} Zellkulturschalen. Außer der VSVG-ECFP exprimierenden Zelle (Pfeilspitze) zeigen zwei weitere, mit dieser über TNTs verbundenen Zellen (Sterne) eine Plasmamembranfärbung. Man beachte, dass auch die TNTs zwischen den Zellen stark mit VSVG-ECFP gefärbt sind (Pfeile). Daraus folgt, dass virales VSVG-ECFP über die TNT-Verbindung zwischen Zellen transferiert wurde, (Balken: 20 Mikrometer);
- Fig. 17e, f: Transfer von HLA-A2-EGFP, einer Komponente des MHC I-Komplexes. Abbildung gemäß Fig. 3f-i2, jedoch wurden die Zellen von Population 1 mit HLA-A2-EGFP transfiziert.Man beachte, dass HLA-A2-EGFP selektiv in über TNT-verbundenen Zellen von Population 2 nachgewiesen wurde (Pfeilspitzen), d. h. zwischen TNT-verbundenen Zellen transferiert wurde.

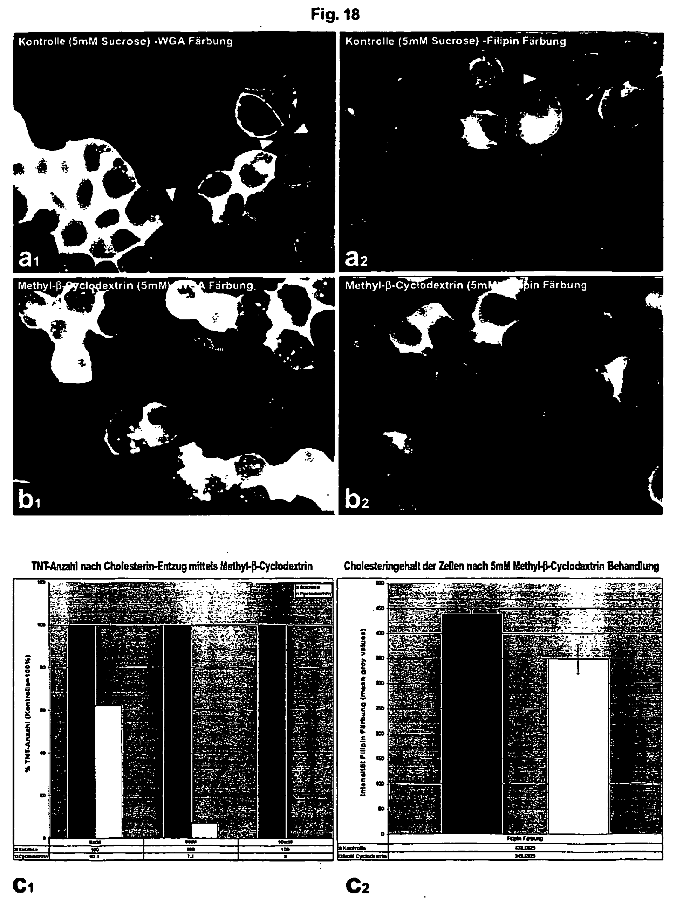

- Fig. 18: den Effekt von Cholesterin auf die Stabilität von TNTs. Zwei Stunden nach Passage von PC12-Zellen wurde das Zellkulturmedium gegen DMEM mit 5, 8 oder 10 Millimolar Saccharose (Kontrollen) oder DMEM mit 5, 8 oder 10 mM Methyl-β-cyclodextrin ausgetauscht und die Zellen für eine halbe Stunde bei 37° C und 10% CO₂ inkubiert. Nach Zugabe von WGA wurde eine dreidimensionale Analyse von 10 zufällig ausgewählten mikroskopischen Feldern durchgeführt (Olympus IX70, 100x Objectiv, TillvisION System, Piezo-z-stepper, je 40 Schnitte) und die Anzahl der TNTs bestimmt. (a1, b1) zeigen für die 5 Millimolar Methyl-β-cyclodextrin-Bedingung representative Bildebenen der dreidimensionalen Analyse, in denen TNTs mit Pfeilspitzen markiert sind. Die Quantifizierung ergab eine starke Abnahme der TNT-Anzahl mit steigender Methyl-β-cyclodextrin Konzentration: 40% Abnahme bei 5 Millimolar Methyl-β-cyclodextrin; 93% Abnahme bei 8 Millimolar Methyl-β-cyclodextrin; 100% bei 10 Millimolar Methyl-β-cyclodextrin (c1). Um den Effekt von Methyl-β-cyclodextrin zur Senkung des zellulären Cholesteringehalts nachzuweisen, wurde eine Filipin-Färbung (Sigma, F-9765) durchgeführt. Dazu wurde das Methyl-β-cyclodextrin- bzw. Saccharose-haltige DMEM der analysierten Zellen mit DMEM mit 20 Mikrogramm pro Milliliter Filipin ausgetauscht und 15 Minuten später die Intensität der Fluoreszenzfärbung *in vivo* mit einem FITC/TRITC/DAPI-Filtersatz (Chroma, Brattleboro) analysiert. Im Detail wurden nach Anregung mit 400 Nanometer vier zufällig ausgewählte mikroskopische Felder aufgenommen und die mittleren Graustufenwerte bestimmt. Die Auswertung nach Behandlung mit 5 Millimolar Methyl-β-cyclodextrin ergab eine Reduktion des zellulären Cholesteringehalts um 20% (c2);

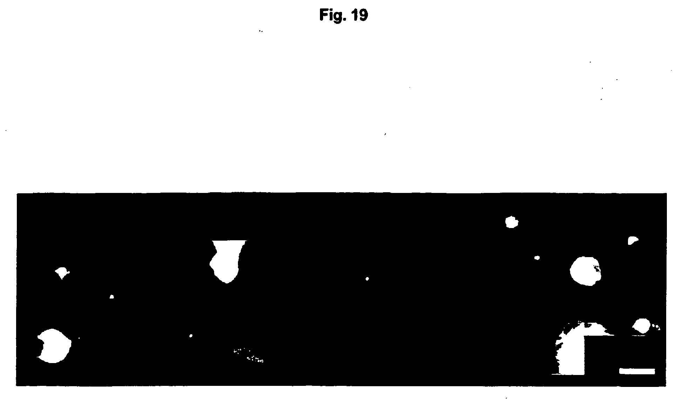

- Fig. 19: eine fluoreszenzmikroskopische Aufnahme einer extrem langen und extrem sensitiven TNT-ähnlichen Struktur zwischen zwei PC12-Zellen. PC12-Zellen wurden in einer LabTek-Kammer durch vorsichtige Zugabe (lokale Applikation) von Dil (Molecular Probes, Oregon) zum Zellkulturmedium gefärbt. Während der Färbung und der simultan stattfindenden mikroskopischen Analyse wurden, soweit möglich, destruktive Energieformen (Erschütterungen, Bewegungen des Mediums, zu starke Lichteinstrahlung) vermieden. Man beachte den feinen, ca. 300 µm langen Membranfaden mit einem apparenten Durchmesser von ca. 200 nm der zwischen den Zellen aufgespannt ist, ohne das Substrat zu berühren, (Balken: 20 µm);

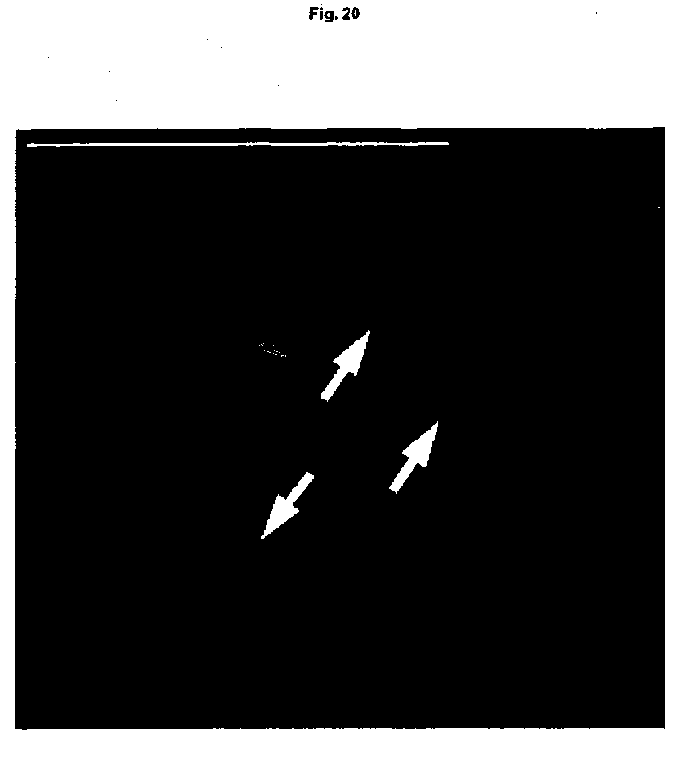

- Fig. 20: eine Mehrfarben-Videomikroskopieaufnahme von einer WGA-gefärbten PC12-Zelle 24 Stunden nach Transfektion mit einer cDNA, die grünfluoreszierendes humanes Chromogranin B-EGFP kodiert [Kaether, C, Salm, T., Glombik, M., Almers, W. & Gerdes, H.-H.: Targeting of green fluorescent protein to neuroendocrine secretory granules: a new tool for real time studies of regulated protein secretion, in Eur. J. Cell Biol. 1997, 74:133-142) - ein für sekretorische Granula charakteristisches Markerprotein, so dass in der Aufnahme die sekretorischen Granula (siehe Pfeile - hellgrau in der Schwarzweiß-Abbildung) grün fluoreszierend sind und rot gefärbt (mittelgrau) die Plasmamembranen der Zellen sowie die neu entdeckten Zelltuben - die Häufungen der sekretorischen Granula an den Kontaktzonen TNT-verbundenen Zellen legt nahe, dass die TNTs an der regulierten Hormon- und Neuropeptidsekretion beteiligt sind, (Balken: 20 Mikrometer);

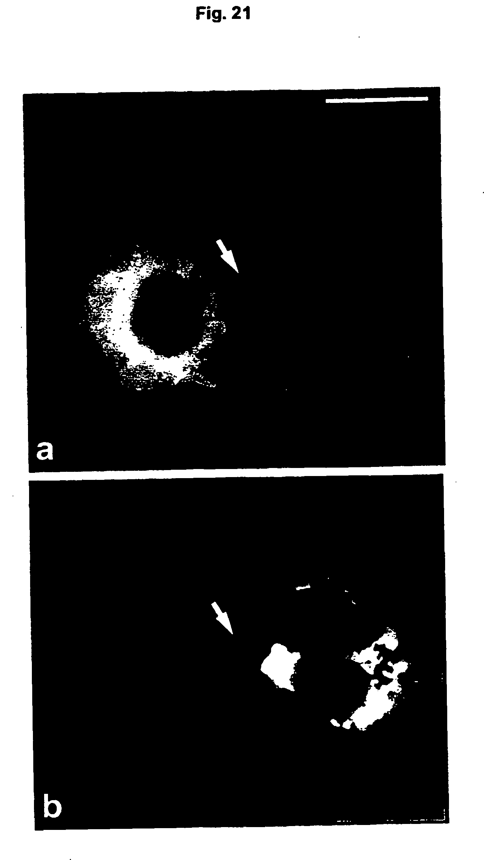

- Fig. 21:: Weder sekretorische Granula noch Mitochondrien werden zwischen TNT-verbundenen Zellen ausgetauscht. Abbildung gemäß Abbildung 3f-i2, jedoch wurden die Zellen von Population 1 mit Chromogranin-B-EGFP (a) oder EYFP-Mito (b, pEYFP-Mito, Clontech 6115-1) transfiziert. Man beachte, daß in den über TNTs (Pfeile) verbundenen Zellen von Population 2 keine Signale der verwendeten Markerproteine gefunden wurden;

### Eingehende Beschreibung der Erfindung und ihre Abführung

Bei der Untersuchung der Proteinsekretion in einer neuroendokrinen Zelllinie wurden bei der dreidimensionalen videomikroskopischen Untersuchung der Zellkultur tubenartige Transportkanäle beobachtet, welche benachbarte Zellen über zum Teil sehr große Entfernungen von mehreren Zelldurchmessern verbrücken (Fig. 1). Die Tubenstränge wurden nach Anfärbung der Plasmamembran mit fluoreszenzmarkiertem Weizenkeim-Agglutinin (WGA - "wheat germ agglutinin") zufällig während der Einstellung der Fokusebene am Fluroeszenz-Videomikroskop beobachtet (Fig. 1a-c). Die Tuben sind im Wesentlichen geradlinig und frei zwischen den Zellen aufgespannt, als ob sie unter Zug stünden, und sind zumeist nicht in Kontakt mit dem Substrat (Fig. 1d). Ihre Architektur unterscheidet sich von allen bisher bekannten Zellfortsätzen. Die Tuben sind in der Regel bis zu 50 Mikrometer lang und besitzen einen mittleren Durchmesser von etwa 200 Nanometer, der jedoch in einigen Fällen bis zu 400 nm und mehr sein kann. Sie werden daher im Folgenden Transport-Nanotuben oder kurz TNTs genannt. Morphologisch sehr ähnliche Strukturen, die mehr als ein Millimeter lang waren, wurden nach einer modifizierten Anfärbemethode beobachtet (siehe Fig. 19). Die TNTs sind sehr empfindlich gegenüber elektromagnetischen Wellen wie Licht (Fig. 9), mechanischen Einwirkungen (Schallwellen) und chemischer Fixierung. Jedoch wurde keine Empfindlichkeit der TNTs gegenüber Trpysin/EDTA-Behandlung festgestellt (Fig. 10), einer Prozedur, die eine proteinvermittelte Zell-Zelladhäsion zerstört. Die immunohistochemische Analyse zeigt, dass die TNTs filamentöses Aktin (F-Aktin) aber keine Mikrotubuli enthalten (Fig. 1e, Fig. 6d1-d3); ähnlich wie Cytoneme von Drosophila-Imaginal-Diskzellen (Ramirez-Weber F.A. et al., Cytonemes: cellular processes that project to the principal signaling center in Drosophila imaginal discs. Cell 97, 599-607 (1999)). Während für Cytoneme eine gerichtete Diffusion von Signalstoffen, z. B. von Morphogenen postuliert wurde, konnten wir mit Ausnahme von Aktin (Fig. 3j-m2) nicht feststellen, dass in TNTs bspw. cytoplasmatisch exprimierte rekombnante Proteine (bspw. EYFP, Fig. 7) oder kleine Farbstoffmoleküle (bspw. Calcein, Fig. 13d1-f2) weitergeleitet werden. Der geringe Innendurchmesser der TNTs verhindert vermutlich einen passiven Austausch von löslichen Molekülen. Dies gilt vermutlich auch für Ionen des Zytoplasmas. Dagegen können über TNTs Komponenten der Zellplasmamembran zwischen den Zellen ausgetauscht werden. So konnten wir an fixierten und lebenden Zellen zeigen, dass farnesyliertes EGFP, ein sehr spezifischer Plasmamembranmarker (Fig. 8), selektiv zwischen über TNTs verbundenen Zellen ausgetauscht wird (Fig. 3n-q2, 4a-c). Daraus folgt, dass die Plasmamembranen der entsprechenden Zellen ein Kontinuum darstellen.

Ferner haben wir gefunden, dass in TNTs ein prominenter Transport von Membranvesikeln von einer Zelle zur anderen erfolgt. Wir konnten mittels der neuen Verfahren unter dem Lichtmikroskop beobachten, dass in den zwischen den Zellen aufgespannten TNTs Vesikel unidirektional mit einer Geschwindigkeit von 25,9 ± 7,9 Nanometer pro Sekunde (n = 6) transportiert werden (Fig. 2g, h). Da TNTs auch bei kultivierten Nieren-Epithelzeilen (Vero-Zellen), humanen embryonalen Nierenzellen (HEK, Fig. 16a) als auch in Primärkulturen von Medulla- (Fig. 16b) und Hippocampus-Gewebe (Fig. 16c) nachgewiesen werden können, sind sie ein generelles Transport-, Kommunikations- und Interaktionsprinzip zwischen Zellen. Der interzelluläre Transport von Membranstrukturen zwischen Zellindividuen über Nanotuben war bislang nicht bekannt. Weiterhin stellt die auf Organellentransport basierende Zell-Zell-Kommunikation ein neues biologisches Funktionsprinzip dar, das von entscheidender Bedeutung für die Entwicklung und Aufrechterhaltung multizellulärer Organismen ist. Daraus ergeben sich wirtschaflich verwendbare Verfahren z. B. für die medizinische Diagnostik und Therapie.

Die hochsensiblen TNTs, welche in dissoziierten Zellkulturen nachgewiesen wurden, existieren vermutlich auch unter physiologischen Bedingungen im Gewebsverband. Wahrscheinlich ist, daß sie dort in ihren strukturellen und funktionellen Charakteristika sehr ähnlich sind, jedoch auf Grund der örtlichen Gegebenheiten eine andere Architektur haben. Eine veränderte Architektur von TNTs (nicht aufgespannt und den Zellkonturen folgend) zeigt sich bereits in konfluenten Verbänden kultivierter Zellen (Vergleiche Fig. 17). TNTs wurden bislang als solche nicht erkannt bzw. beobachtet, weil sie erstens sehr empfindlich sind und in Zellkulturen in der Regel durch die mikroskopischen Standardverfahren, beispielsweise durch die mechanische Energie der Waschschritte, chemische Fixierungsmethoden oder nur durch die Energie des eingestrahlten Lichtes zerstört werden (Vergleiche Fig. 9). Zweitens, weil bei nicht optimalen Zellkulturbedingungen und ungeeigneter Zelldichte die Anzahl der frei zwischen Zellen aufgespannten TNTs gegen Null geht. Drittens, weil die zwischen den Zellen aufgespannten TNTs in einer für die meisten anderen mikroskopischen Studien ungeeigneten und damit vernachlässigten Ebene der Zellen liegen. Viertens, weil TNTs oft nicht horizontal, sondern mit einer Neigung zum Zellkulturgefäß verlaufen, wodurch sie nicht von Anfang bis Ende in einer mikroskopischen Ebene sichtbar sind, d. h. nur über eine dreidimensionale Analyse nachgewiesen werden können.

Viele Pathogene sind in ihrem Vermehrungs- und Verbreitungszyklus auf den interzellulären Transfer zwischen Zellen angewiesen. In sehr vielen Fällen sind die genauen Mechanismen dieses Transfers noch nicht aufgeklärt worden. So wird z.B. das virale HSV1 Tegumentprotein VP22 extrem effizient zwischen Zellen ausgetauscht (Wybranietz, W. A. et al. Enhanced suicide gene effect by adenoviral transduction of a VP22-cytosine deaminase (CD) fusion gene. Gene Therapy 8, 1654-1664 (2001)). Auch an VP22 angehängte Proteine unterliegen diesem Austausch, was VP22 zu einem wichtigen Werkzeug für gentherapeutische Verfahren macht (Wybranietz, W. A. et al. Enhanced suicide gene effect by adenoviral transduction of a VP22-cytosine deaminase (CD) fusion gene. Gene Therapy 8, 1654-1664 (2001). Es ist bisher jedoch ungeklärt , wie VP22 bzw. VP22-Fusionsproteine von einer zur anderen Zelle gelangen. Wir nehmen an, daß die hier beschriebenen TNTs von Pathogenen als Infektionsweg benutzt werden können. Gestützt wird unsere Annahme durch Experimente, in denen wir zeigen konnten, daß die viralen Proteine VP22 (Fig. 17a-c) und VSVG (Fig. 17d) in TNTs lokalisiert sind, dort vesikulär transportiert werden und zwischen den verbundenen Zellen transferiert werden. Eine weitere Evidenz ist die Beobachtung anderer, daß VP22 mit Aktin interagieren kann und der interzelluläre Austausch dieses **Proteins** durch Zerstörung des filamentösen Aktins durch Cytochalasin D unterbunden werden kann (Elliott, G. & O'Hare, P. Intercellular trafficking and protein delivery by a herpesvirus structural protein. Cell 88, 223-233. (1997)). Zusammenfassend werden TNTs und ihre Beeinflussung damit zu wichtigen Ansatzstellen z. B. zur Verbesserung oder Schaffung neuer gentherapeutischer Verfahren sowie zur Einflußnahme und Eindämmung infektiöser Erkrankungen.

Die neu entdeckten Zell-Zell-Verbindungen können wegen ihrer fadenartigen Natur und Labilität in vielfältiger Form beeinflusst werden: (1) durch Schall, Vibrationen, Hitze oder hydromechanische Energieformen und auch (2) durch Licht und andere elektromagnetische Wellen (Vergleiche Fig. 9). Weiterhin können komplexe pathologische Körperfunktionen wie Bluthochdruck über das TNT-Konzept (Fig. 5) mit subtilen Umwelteinflüssen wie elektromagnetischer Strahlung, Lärm und so weiter mit einem objektiven zellbiologischen Transport- und Interaktionsvorgang in Verbindung gebracht werden. Ferner kann (3) durch pharmakologisch wirksame Substanzen wie z. B. Methyl-β-cyclodextrin, welches Cholesterin aus Zellmembranen extrahiert, gezielt die Lipidzusammensetzung und damit die Eigenschaften der Membranen verändert werden. Diese Beeinflussung wirkt sich nachhaltig auf die Stabilität der TNTs aus. So führt eine Senkung des zellulären Cholesteringehalts um 20% zu einer 40%-igen Reduktion der TNT-Anzahl (Fig. 18). Die Erfinding stellt somit ein Modell bereit, TNT-vermittelte Zellkommunikation fein zu regulieren und ggfs. pathologisch bedingte Veränderungen zu korrigieren. Zudem erlauben (4) klassische biochemische Einflüsse wie die Veränderung ihres Aufbaus, der Zusammensetzung oder die Fehlregulierung ihrer Bildung durch Krankheit, zum Beispiel durch Stoffwechselkrankheiten, oder durch Medikamente wichtige diagnostische Aussagen, insbesondere im Bereich der psychischen Erkrankungen und psychogenen Medikamente, da hier Kommunikationsvorgänge erwartungsgemäß eine große Rolle spielen. Die Erfindung stellt somit auch ein Modell bereit zur Untersuchung, Prüfung und zur Auffindung neuer pharmazeutischer Wirkstoffe. Daneben zeigen die gefundenen TNTs verblüffende morphologische Gemeinsamkeiten mit *in vitro* hergestellten Nanotuben aus Phospholipid-Doppelmembranen (Karlsson, A. et al., Networks of nanotubes and containers, Nature 409, 150-152 (2001)). Auch bei diesen *in vitro* erzeugten Nanotuben wird ein Membrankontinuum zwischen den Liposomen hergestellt und es kann ein Transport von Lipidbehältem über die Nanotuben erfolgen. Es liegt nahe, das gefundene Transportprinzip für *in vitro* hergestellte Nanotuben bzw. Liposomen und das Medikament-Targeting zu nutzen.

Die aufgefundenen TNTs sind frei zwischen den Zellen über die kürzeste Strecke gespannte, von einer Membran umgebene, elastische fadenartige Kanäle, die in der Regel keinen Kontakt zum Substrat, beispielsweise der Zellkulturplatte haben (Fig. 1). Ihr Durchmesser liegt in der Größenordnung von weniger als 200 Nanometer, kann jedoch in einigen Fällen bis 400 Nanometer erreichen. Da TNTs nicht auf dem Substrat aufliegen und wegen ihrer Länge in der Regel mehrere Fokusierebenen des Mikroskops kreuzen, sind sie in herkömmlichen Mikroskopieverfahren nicht direkt als interzelluläre Tuben erkennbar, sondern werden nur als unspezifische Fortsätze oder Zellröhren ohne Ausgang und Ziel gesehen. Die Länge der neuen interzellulären Verbindungen (TNTs) beträgt meistens bis zu 50 Mikrometer (Fig. 1) und gelegentlich auch bis zu über einem Millimeter (Fig. 19). Vereinzelt konnten auch verzweigte TNTs beobachtet werden (siehe Fig. 1c, Pfeil).

TNTs enthalten F-Aktin, jedoch keine Mikrotubuli (Fig. 1e, Fig. 6d1-d3). Das F-Aktin ist vermutlich wegen seines hohen Anteils in TNTs auch an der Ausbildung dieser Zellverbindung strukturell und funktionell beteiligt (Vergleiche Fig. 2a-d). Diese Vermutung wird gestützt durch die Beobachtung, daß in Gegenwart von Latrunculin B, welches F-Aktin depolymerisiert, keine TNTs mehr gebildet bzw. vorhandene TNTs zerstört werden (Fig. 11b). Wenngleich gelegentlich punktierte Signale von Tubulin in TNTs zu beobachten sind, so besitzen diese nicht die für Mikrotubuli charakteristische Filamentstruktur (Fig. 1e). Weiterhin wurde immuncytochemisch in TNTs punktförmig Synaptophysin, ein Membranmarker für "small synaptic-like microvesicles" (SLMVs, Hannah M.J. et al., Synaptic vesicle biogenesis, Annu. Rev. Cell Dev. Biol., 15, 733-798 (1999)) nachgewiesen (Fig. 3c, e). SLMVs enthalten Signalmoleküle wie Acetylcholin (Bauemfeind R. et al., Selective storage of acetylcholine, but not catecholamines, in neuroendocrine synaptic-like microvesicles of early endosomal origin, Neuron 11, 105-121 (1993)), so daß TNTs an der Signalweitergabe zwischen Zellen beteiligt sind. Ferner wurde Myosin Va (Fig. 3d, e), ein aktinabhängiges Motorprotein und Rab3a (Fig. 3e2), ein monomeres GTP-bindendes Protein in TNTs gefunden. Es konnte auch eine partielle Kolokalisation von Myosin Va und Synaptophysin-positiven Organellen in TNTs festgestellt werden (Fig. 3e, offener Pfeil). Der sichtbare Organellentransport sowie die gleichzeitige Anwesenheit von F-Aktin, Myosin Va und GTP-bindender Proteine deuten auf einen Aktin bzw. Myosin vermittelten Organellentransport hin (Mermall V. et al., Unconventional myosins in cell movement, membrane traffic, and signal transduction, Science 279, 527-533 (1998)). Zudem wurde gefunden, daß sekretorische Granula, Hormon speichernde Organellen, *in vitro* und *in vivo* an der Basis von TNTs gehäuft vorliegen (Fig. 20, Pfeile). Dies läßt auf einen funktionellen Zusammenhang zwischen TNTs und dem endokrinen System schließen.

TNTs sind sehr empfindliche Strukturen. Geringer mechanischer Stress, chemische Fixierung oder wellenförmige Energieformen wie z. B. wenige Sekunden Lichtbestrahlung mit einer Wellenlänge von 565 Nanometer lässt sie in vielen Fällen reißen (Fig. 9). Dagegen sind TNTs im Unterschied zu Filopodien oder Axonen unempfindlich gegenüber Trypsinbehandlung (Fig. 10). TNTs bildeten sich rasch *de novo* zwischen Zellen und waren bereits 30 Minuten nach der Ausplattierung von PC12-Zellen zu beobachten (Fig. 2e, f, Fig. 11a). Ihre Anzahl nahm dann innerhalb der folgenden 1,5 Stunden stark (8-fach) zu (Fig.11a). Da eine Blockierung der Zellteilung (G1/S-Phasen-Block) in dieser Zeit ihre Bildung nicht beeinflusst (Fig. 12), sind TNTs kein Produkt einer unvollständig verlaufenden Zytokinese.

Im Gegensatz zu gängigen kommunikativen Zellverbindungen wie Plasmodesmata oder "Gap-Junctions" zeigen TNTs kaum messbare passive Durchlässigkeit für kleine mikroinjizierte Farbstoffmoleküle wie z. B. Calcein (Fig. 13) oder Bodipy. Ebenfalls wurde kein signifikanter Transfer von cytoplasmatisch exprimiertem EYFP bzw. ECFP beobachtet (Fig. 7). Dagegen wurde Aktin, eine strukturelle Komponente der TNTs, in Form eines GFP-Fusionsproteins (EGFP-Aktin) selektiv zwischen TNT-verbundenen Zellen transferiert (Fig. 3j-m2). Ebenfalls wurde ein TNT-vermittelter, interzellulärer Austausch von famesyliertem EGFP, einem spezifischen Marker für die Plasmamembran (Fig. 8), nachgewiesen (Fig. 3n-q2). Als einzigartiges Merkmal von TNTs wurde ein unidirektionaler Transfer von Membranvesikeln durch diese Strukturen gefunden. Diese Vesikel waren positiv für Synaptophysin-EGFP (Fig. 3c, f-i2), für Lysotracker™ (Fig. 3a, b) oder für die endozytierten Farbstoffe Dil oder DiO (Honig et al., Dil and DiO: versatile fluorescent dyes for neuronal labelling and pathway tracing. Trends Neurosci., 12, 331-340; Kuffler D.P., Long-term survival and sprouting in culture by motoneurons isolated from the spinal cord of adult frogs. J. Comp. Neurol., 302, 729-738 (1990)) (Fig. 4d-h). Der TNT-vermittelte, unidirektionale Transfer von Dil oder DiO markierten Organellen von einer Zelle zur anderen konnte über Fluoreszenzvideomikroskopie direkt beobachtet werden (Fig. 4d, e, Fig. 14). Zusätzlich konnte bereits zwei Stunden nach Anlegen einer Mischkultur aus Dil- bzw. DiO-gefärbten Zellen konnte beobachtet werden, daß unterschiedliche Zellen über TNTs die grün bzw. rot fluoreszierenden Organellen untereinander unidirektional austauschten (Fig. 4g1). Dieser Austausch wurde nachweislich nicht über Mechanismen bewerkstelligt, die gängige Endo- und Exozytosemechanismen mit einschließen. Diese Annahme wird durch Transferexperimente um 0° C gestützt, einer Temperatur, die effizient bekannte Endo-und Exozytosemechanismen blockiert. Unter diesen Bedingungen fand ein im Vergleich zu 37° C reduzierter, jedoch weiterhin signifikanter Organellentransfer statt (Fig. 15). Dieser Befund unterstützt das Modell der über TNT-Verbindungen gebildeten Membrankontinuität zwischen den Zellen (Fig. 5).

In TNTs konnten außerdem Transportvorgänge, die einem vesikulären Transport gleichen, über Hellfeld-Videomikroskopie nachgewiesen werden (Fig. 2g, h). Hellfeld- bzw. Fluoreszenzmikroskopie zeigten einen unidirektionalen Transport dieser Strukturen von Zelle zu Zelle mit Geschwindigkeiten von 8 bis 100 Nanometer pro Sekunde (Fig. 2gh, Fig. 3ab). Diese Ergebnisse zusammen mit dem Nachweis Synaptophysin-positiver Strukturen (Fig. 3c) oder Lysotraker^{™}-positiver Strukturen (Fig. 3a, b) legen nahe, daß es sich bei den von Zelle zu Zelle transportierten Strukturen zumindest teilweise um SLMVs und/oder Membranstrukturen handelt, die zum endosomalen System gehören oder daraus hervorgegangen sind. Die Kolokalisation dieser Strukturen mit Myosin Va (Fig. 3e) zeigt, dass wahrscheinlich Myosin Va partiell am Transport dieser Strukturen und Rab3a an der Regulation des Transports beteiligt sind. Sekretorische Granula und Mitochondrien wurden dagegen nicht in TNTs beobachtet bzw. wurden nicht signifikant zwischen den Zellen ausgetauscht (Fig. 21). Diese Organellen werden also vermutlich über Größenselektion oder andere bisher unbekannte Mechanismen vom TNT-vermittelten Transport ausgeschlossen.

TNTs wurden nicht nur für die PC12-Zelllinie aus dem Nebennierenmark von Ratten gefunden, sondern konnten auch in einer Nierenepithelzelllinie der grünen Meerkatze (Vero-Zelllinie) und humanen HEK-Zellen (Fig. 16a) sowie in Primärkulturen der Ratte gewonnen aus Medulla (Fig. 16b) und Hippocampus (Fig. 16c) nachgewiesen werden. Somit treten TNTs in Zellen auf, die aus höchst unterschiedlichen Gewebstypen stammen. Darüber hinaus belegen die Ergebisse, daß TNTs nicht nur in gesundem, sondern auch in Krebsgeweben vorkommen. Es liegt somit nahe, daß gewebstypspezifische TNTs mit spezifischen Eigenschaften existieren. Es ist zu erwarten, daß TNTs insbesondere auch während der Embryonalentwicklung von den Zellen ausbildet werden und eine wichtige Rolle bei z. B. der Musterbildung haben. Es liegt somit nahe, daß TNTs, erstens, für bestimmte Formen der Zell-Zell-Kommunikation verantwortlich sind, zum Beispiel für die Gewebsbildung und den -erhalt; zweitens, in kausalem Zusammenhang stehen mit verschiedenen Erkrankungen wie z. B. Krebs oder viralen Infektionen, indem sich virale Proteine wie z. B. VP22 oder das VSV G-Protein über TNTs im Gewebe verbreiten (Fig. 17a-d); drittens, wegen ihrer hohen Sensibilität gegenüber äußeren Energieformen leicht beeinflussbar und/oder zerstörbar sind (z. B. durch Licht (Fig. 9)) und auf diese Weise Einfluss auf komplexe Körperfunktionen nehmen; viertens, gezielte therapeutische Ansatzpunkte für viele Krankheiten darstellen, die z. B. auf das durch TNTs aufgespannte zelluläre Netzwerk und/oder deren Sensibilität basieren. In diesem Zusammenhang wurde nachgewiesen, daß der Entzug von Cholesterin aus Zellmembranen TNTs zerstört oder ihre Neubildung verhindert (Fig. 18) und zudem eine präzise Regulation ihrer Ausbildung über eine Einstellung des Cholesteringehalts von Membranen möglich ist (Fig. 18). Als Krankheiten seien hier beispielhaft Krebs und Bluthochdruck genannt. Es ergeben sich somit unmittelbar folgende Mittel und therapeutische Anwendungen zur Behandlung von Erkrankungen.

### BEISPIELE

### Beispiel 1 Mittel zur Therapie von Tumoren

### 1. Beeinflussung von TNTs durch externe wellenförmige Energieformen

In Krebszellen wie z. B. der PC12-Zelllinie aus Pheochromocytoma-Gewebe wurden TNTs nach Färbung mit WGA durch hochauflösende, dreimensionale Videomikroskopieaufnahme circa 24 Stunden nach Zellpassage gemäß Beispiel 15 nachgewiesen (Fig. 1a-d). An diesen Zellen wurde der Einfluß von externen Energieformen wie z. B. von sichtbarem Licht eines mikroskopischen Systems (gemäß Beispiel 15 Punkt 2) mit einer Wellenlänge von 565 Nanometer getestet. Über einen Beobachtungszeitraum von ca. 70 Sekunden wurde gezeigt, dass die TNT- Verbindung (Fig. 9a) von dem einfallenden Licht der Wellenlänge 565 nm zunächst in Schwingung versetzt wird (Fig. 9b), reißt (Fig. 9c) und sich innerhalb Sekunden an seinem losen Ende wie ein gerissenes Gummiband aufwickelt (Fig. 9d). Ein ähnlicher Effekt auf TNTs ist auch von anderen wellenförmigen Energieformen zu erwarten. Wegen dieser Empfindlichkeit von TNTs gegenüber externen Energieformen bietet es sich an, insbesondere Pheochromocytoma-Tumore mit Infra- oder Ultraschall bzw. Licht- oder Magnetfeldtherapie zu behandeln. Für diese Behandlung kommen kommerziell erhältliche bzw. speziell für diese Applikation entwickelte Generatoren für z. B. gepulste Magnetfelder in Frage. Die TNTs zwischen den Tumorzellen können so gezielt geschädigt werden. Da auch gesundes Gewebe interzelluläre Fadenverbindungen ausbilden kann, sollte die Applikation dieser Energieformen gezielt und lokal erfolgen, um eine selektive Schädigung des Tumorgewebes zu erreichen. Die Nebenwirkungen einer solchen Behandlung wären vermutlich erheblich niedriger als eine entsprechende radioaktive Bestrahlung oder der Einsatz von ChemoTherapeutika. Weiterhin stellen PC12-Zellkulturen und ggfs. chromaffine Primärkulturen gemäß Beispiel 16 geeignete Modellsysteme dar, um die für diesen Therapieansatz effektivsten und selektivsten Frequenzen und Intensitäten der verwendeten Energieformen zu ermitteln.

### 2) Gezieltes "targeting" von Zytopharmaka oder anderer Komponenten

### a) Targeting über TNT-vermittelten Transfer von Organellen

In Krebszellen wie z. B. der Zelllinie PC12 aus Pheochromocytoma-Gewebe wurde mittels hochauflösender, dreimensionaler Durchlichtmikroskopie circa 24 Stunden nach Zellpassage (gemäß Beispiel 15) nachgewiesen, daß ein auf TNTs basierendes interzelluläres Netzwerks *de novo* gebildet wird (Fig. 2a-f). Weiterhin konnte mit direkter Hellfeldvideomikroskopie (gemäß Beispiel 15 Punkt 2) der unidirektionale, TNT-vermittelte interzelluläre Organellentransfer beobachtet werden (Fig. 2g,h). Fluoreszenz-Videomikroskopieaufnahmen (gemäß Beispiel 15 Punkt 2) dieser Zellen nach Färbung mit Lysotracker^{™} (gemäß Beispiel 15 Punkt 5) zeigen, daß fluoreszierende Organellen unidirektional in TNTs transferiert werden (Fig. 3a, b) und wahrscheinlich endosomalen Ursprungs sind. Fluoreszenz-Videomikroskopieaufnahmen (gemäß Beispiel 15 Punkt 2) von PC12-Zellen nach Antikörper-Färbung von Synaptophysin, Myosin Va und Rab 3a (gemäß Beispiel 15 Punkt 4) sowie TRITC-Phalloidin-Aktinfärbung (gemäß Beispiel 15 Punkt 4) zeigen, daß Synaptophysin- und Myosin Va-Signale an derselben Stelle in TNTs zu finden waren (Fig. 3c-e2). Diese jeweils punktförmigen Färbungen in Verbindung mit der direkten Beobachtung des Organellentransfers (Fig. 4d, e, Fig. 14) zeigen, dass in TNTs Synaptophysin-positive Organellen (z. B. SLMVs) von Motorproteinen wie z. B. Myosin Va fortbewegt werden und regulatorische Proteine wie z. B. Rab3a an diesem Transport beteiligt sind.

Der hier gezeigte selektive Austausch endosomaler bzw. endosomenverwandter Organellen zwischen TNT-verbundenen Zellen stellt ein effizientes Transportsystem dar, über das Zytopharmaka bzw. andere Komponenten in Gewebsverbänden wie z. B. Tumoren gezielt und kontrolliert verbreitet werden könneri. Zur Demonstration wurde exemplarisch EGFP gentechnisch an Synaptophysin, welches selektiv mit Endosomen und damit verwandten Organellen assoziiert ist, gekoppelt (Synaptophysin-EGFP). Der Transfer dieses Fusionsproteins zwischen Krebszellen wurde durch folgenden Versuchsablauf dokumentiert. Eine Population von PC12-Zellen wurde (gemäß 15 Punkt 7) mit CellTracker™ (Molecular Probes, "blue" C-2110) gefärbt (Population 2) und mit einer weiteren Population PC12-Zellen, die mit Synaptophysin-EGFP transfiziert wurde (Population 1) gemischt ausplattiert. Die gemischten Zellkulturen wurden 24-48 Stunden später fixiert und immuncytochemisch mit einem polyklonalen Anti-GFP Antikörper (Molecular Probes A-6455) und Phalloidin-FITC gefärbt (gemäß Beispiel 15 Punkt 4). Die Zellen wurden dann mittels dreidimensionaler Fluoreszenzmikroskopie und Dekonvolution der aufgenommenen Bilddaten gemäß Beispiel 15 Punkt 2 analysiert. Synaptophysin-EGFP wurde in Form punktierter Signale selektiv in TNT-verbundenen Zellen von Population 2 nachgewiesen (Fig. 3f-i2), d. h. selektiv zwischen diesen Zellen transferiert. Über eine Analyse mit Dil/DiO gefärbten Organellen gemäß Beispiel 14 konnte nachgewiesen werden, daß TNTs den interzellulären Transfer von Dil/ DiO gefärbten Organellen nach einem Mechanismus bewerkstelligen, der Aktin-abhängig ist, aber gängige endo-und exozytotische Mechanismen ausschließt. Dazu wurden zwei unterschiedlich gefärbte PC12-Zellpopulationen als Mischkultur auf LabTek^{™}-Kulturschalen für 1 Stunde bei 37° C kultiviert und dann bei verschiedenen Temperaturen in Abwesenheit oder in Gegenwart von 5 Mikromolar Latrunculin-B weiter kultiviert. Zellkulturen ohne Latrunculin-B, die 1 Stunde nach Ausplattierung mit WGA gefärbt, anschließend für weitere 4 Stunden bei 0.7° C oder 37° C kultiviert, fixiert und dann einer videomikroskopischen Analyse zugeführt wurden, zeigten beide einen deutlichen, TNT-abhängigen Organellentransfer (Fig. 15a, e), obwohl der Transfer bei 0.7° C, einer Temperatur die gängige Endozytosemechanismen blockiert (Fig. 15, vergleiche c und d), niedriger war (Fig. 15e). Ein im Tumorgewebe existierendes und auf TNTs basierendes zelluläres Netzwerk eröffnet daher die Möglichkeit, durch gezieltes Einbringen von toxischen, apoptotischen oder immunogenen Wirkstoffen (Substanzen bzw. gentechnisch hergestellte Proteine, die gemäß dem oben beschriebenen Beispiel mit den nötigen Signalen für den Transport über TNTs ausgestattet sind), die durch TNTs verbundenen Zellen gezielt zu zerstören oder regulativ zu beeinflussen z. B. gemäß Beispiel 1 Punkt 1.

### b) Targeting über den TNT-vermittelten Transfer von Membrankomponenten

Es wurde gezeigt, dass Krebszellen über TNTs ein Syncytium bilden (Fig. 2e, f), über das spezifische Membrankomponenten ausgetauscht werden können. Dieser Nachweis wurde wie folgt durchgeführt. Eine Population von Pheochromocytoma-PC12-Zellen wurde (gemäß Beispiel 15 Punkt 7) mit CellTracker™ (Molecular Probes, "blue" C-2110) gefärbt (Population 2) und mit einer weiteren Population PC12-Zellen, die mit farnesyliertem EGFP (Clontech 6074-1) transfiziert wurde (Population 1) gemischt ausplattiert. Die gemischten Zellkulturen wurden 24-48 Stunden später fixiert und immunzytochemisch mit einem polyklonalen Anti-GFP Antikörper (Molecular Probes A-6455) und Phalloidin-FITC gefärbt (gemäß Beispiel 15 Punkt 4). Die Zellen wurden dann mittels dreidimensionaler Fluoreszenzmikroskopie und Dekonvolution der aufgenommenen Bilddaten (gemäß Beispiel 15 Punkt 2) analysiert. Farnesyliertes EGFP wurde selektiv an der Plasmamembran von mit TNTs verbundenen Zellen von Population 2 nachgewiesen (Fig. 3n-q2), d. h. in Form eines Plasmamembrantransfers selektiv zwischen den TNT-verbundenen Zellen ausgetauscht. Ein im Tumorgewebe existierendes und auf TNTs basierendes zelluläres Netzwerk eröffnet deshalb die Möglichkeit, durch gezieltes Einbringen von toxischen, apoptotischen oder immunogenen Wirkstoffen (Substanzen bzw. gentechnisch hergestellte Proteine, die gemäß dem oben beschriebenen Beispiel mit den nötigen Signalen für den Membrantransfer über TNTs wie z. B. einem Famesylanker ausgestattet sind), die durch TNTs verbundenen Zellen gezielt zu zerstören oder regulativ zu beeinflussen (z. B. gemäß Beispiel 1 Punkt 1).

### Beispiel 2 Mittel zur Therapie von Stoffwechselkrankheiten

Auch Stoffwechselkrankheiten, welche z. B. die Lipidzusammensetzung wie z. B. den Cholesteringehalt von Membranen beeinflussen, haben Auswirkungen auf die Stabilität der gefundenen sensiblen TNTs (Fig. 18, gemäß Beispiel 16 Punkt 1). Deren Abreißen bedingt Mikroläsionen (Fig. 9) spezifisch in den Bereichen der Plasmamembran z. B. neuroendokriner Zellen, wo die Speicherorganellen für Botenstoffe, sogenannte sekretorische Granula, gehäuft vorliegen (Fig. 20) und ist daher ein effizientes Signal für Zellreaktionen wie die Exozytose von Botenstoffen wie Hormonen, Neuropeptiden oder Wachstumsstoffen in die Blutbahn bzw. in den extrazellulären Raum. Das Signal kann auch eine veränderte Exozytoserate bedingen. Dies kann pathologische Effekte haben wie z. B. einen krankhaft erhöhten Hormonspiegel, ist aber auch therapeutisch nutzbar. So kann die Beeinflussung der auf TNTs basierenden Zell-Zell-Kommunikation gemäß Beispiel 16 Signalwirkung haben, die zu veränderten Zellreaktionen führt und nutzbare Mechanismen wie z. B. Apoptose oder Nekrose nach sich zieht. Auch ist durch eine gezielte Einstellung der TNT-Stabilität z. B. durch Veränderung ihrer Lipidzusammensetzung (gemäß Beispiel 16 Punkt 1) oder deren Beeinflussung über externe Energieformen (gemäß Beispiel 16 Punkt 2) eine Feinsteuerung letztgenannter Mechanismen denkbar.

### Beispiel 3 Bluthochdruck

Der Blutdruck wird u. a. durch das hormonelle System wie z. B. über Signalstoffe der Nebenniere kontrolliert. Eine Mehrfarben-Videomikroskopieaufnahme von einer WGA-gefärbten PC12-Zelle der Nebenniere 24 Stunden nach Transfektion (gemäß Beispiel 15) mit einer cDNA, die grün fluoreszierendes humanes Chromogranin B-EGFP kodiert [Kaether, C, Salm, T., Glombik, M., Almers, W. & Gerdes, H.-H.: Targeting of green fluorescent protein to neuroendocrine secretory granules: a new tool for real time studies of regulated protein secretion, in Eur. J. Cell Biol. 1997, 74:133-142] - ein für sekretorische Granula charakteristisches Markerprotein, zeigt die Häufungen der sekretorischen Granula an den Kontaktzonen der TNT-verbundenen Zellen (Abb. 20, Pfeile). Dies legt nahe, dass TNTs an der regulierten Hormon- und Neuropeptidsekretion beteiligt sind. Die Reizung oder Mikroläsion von Nebennierenzellen (z. B. durch Schwingung oder Abreißen von TNTs in diesem Gewebe (gemäß Beispiel 1 Punkt 1 bzw. Beispiel 16 Punkt 2) oder veränderter Lipidzusammensetzung (gemäß Beispiel 16 Punkt 1) kann die Exozytose von Botenstoffen aus Nebennierenzellen in die Blutbahn zur Folge haben. Dadurch kann es zu einem krankhaft erhöhten Adrenalinspiegel kommen, der wiederum zu einem erhöhten Blutdruck führt. Therapieansätze sind gegeben durch Abschirmen bzw. Eliminieren der externen Energieformen durch geeignete Abschirmmaterialien (z. B. durch bereits auf dem Markt befindliche "Wave Shield" als Handy-Strahlenschutz oder spezielle Folien oder Wandfarben von "Protect ES") und/oder durch Beeinflussung von endogenen Faktoren wie z. B. der Lipidzusammensetzung von Zellmembranen. Letzteres kann durch veränderte Ernährung oder Applikation spezifischer Medikamente erreicht werden, wobei dem Cholesterin-Spiegel in diesem Zusammenhang ein besonderer Stellenwert zukommt (Fig. 18, gemäß Beispiel 16 Punkt 1).

### Beispiel 4 Autoimmunkrankheiten

Im Falle von Autoimmunkrankheiten wie z. B. Diabetes Typ I erkennt das Immunsystem körpereigene Proteine. Diese Fehlsteuerung wird oft durch virale Proteine (molekulares Mimikrie) oder falsch gefaltete Proteine ausgelöst. Es wurde gezeigt, dass Zellen über TNTs ein Syncytium bilden, über das Membrankomponenten ausgetauscht werden können. Dieser Nachweis wurde wie folgt durchgeführt. Eine Population von Nebennieren PC12-Zellen wurde mit CellTracker™ (Molecular Probes, "blue" C-2110) gefärbt (Population 2) und mit einer weiteren Population PC12-Zellen, die mit famesyliertem EGFP (Clontech 6074-1) transfiziert wurde (Population 1) gemischt ausplattiert. Die gemischten Zellkulturen wurden 24-48 Stunden später fixiert und immunzytochemisch mit einem, polyklonalen Anti-GFP Antikörper (Molecular Probes A-6455) und Phalloidin-FITC (gemäß Beispiel 15) gefärbt. Die Zellen wurden mittels dreidimensionaler Fluoreszenzmikroskopie und anschließender Dekonvolution der aufgenommenen Bilddaten (gemäß Beispiel 15 Punkt 2) analysiert. Farnesyliertes EGFP, ein spezifischer Marker für die Plasmamembran (Fig. 8), wurde selektiv an der Plasmamembran von mit TNTs verbundenen Zellen von Population 2 nachgewiesen (Fig. 3n-q2), d. h. in Form eines Plasmamembrantransfers selektiv zwischen den TNT-verbundenen Zellen ausgetauscht. Dieses Ergebnis belegt, dass Zelloberflächenproteine über ein im Gewebe existierendes und auf TNTs-basierendes zelluläres Netzwerk verbreitet werden können.

In diesem Kontext konnte auch gezeigt werden, dass TNTs an der Verbreitung von Antigen präsentierenden Oberflächenproteinen, Komponenten der "major histocompatibility" (MHC)-Komplexe in Geweben beteiligt sind. Hierzu wurde eine Population von Pheochromocytoma-PC12-Zellen wurde (gemäß Beispiel 15 Punkt 7) mit CellTracker™ (Molecular Probes, blue" C-2110) gefärbt (Population 2) und mit einer weiteren Population PC12-Zellen, die mit HLA-A2-EGFP (H. J. Geuze, Department of Cell Biology, Institute of Biomembranes, UMC, Utrecht, Holland) transfiziert wurde (Population 1) gemischt ausplattiert. Die gemischten Zellkulturen wurden 24-48 Stunden später fixiert und immunzytochemisch mit einem polyklonalen Anti-GFP Antikörper (Molecular Probes A-6455) (gemäß Beispiel 15 Punkt 4). Die Zellen wurden dann mittels dreidimensionaler Fluoreszenzmikroskopie (gemäß Beispiel 15 Punkt 2) analysiert. HLA-A2-EGFP wurde selektiv in Form punktierter Signale in mit TNTs verbundenen Zellen von Population 2 nachgewiesen (Fig. 17 e, f). Insbesondere der Umstand, dass die MHC - Komplexe über membranöse, zum Teil mit dem endosomalen System verwandte Transporter befördert werden, verdeutlicht die potentielle Beteiligung von TNTs an der Verbreitung dieser Komplexe innerhalb des Gewebes. Aus diesem Grunde kann der Zerstörung des gesamten betroffenen Gewebes dadurch Einhalt geboten werden, dass durch die oben aufgeführten externen Energieformen (gemäß Beispiel 16 Punkt 2) bzw. durch Änderung der Lipidzusammensetzung (gemäß Beispiel 16 Punkt 1) das auf TNTs basierende Netzwerk lokal zerstört oder regulativ beeinflusst wird. Der TNT-vermittelte Transfer immunoreaktiver Komponenten in Gewebsverbänden eröffnet die Möglichkeit, solche Komponenten gezielt in Geweben zu verteilen, d. h. auf diese Weise Immunantworten zu stimulieren, die in letzter Konsequenz zur Elimierung durch das körpereigene Immunsystem führen können.

### Beispiel 5 Prionenkrankheit

Die Prionenkrankheit wird durch ein missgefaltetes Protein (Prion-Protein) ausgelöst, welches über einen Glycosylphosphatidylinositol(GPI)-Anker mit der Zelloberfläche assoziert ist und dadurch infektiös wirkt, indem es eine Missfaltung des korrespondierenden intakten zellulären Proteins induziert. Wie Prionen von Zelle zu Zelle gelangen, ist weitgehend unverstanden. Es wurde gezeigt, dass Nebennierenzellen oder hippocampale Neurone (Fig. 16c) über TNTs ein Syncytium bilden , über das lipidverankerte Membrankomponenten gemäß Beispiel 1 Punkt 2b ausgetauscht werden (Fig. 4a-c, Fig. 3n-q2). Dass über einen vergleichbaren Lipidanker mit der Plasmamembran assoziierte infektiöse Prion-Protein kann deshalb über TNTs auf Nachbarzellen übertragen werden. Therapieansätze sind daher durch Zerstörung des auf TNTs basierenden Neztwerks mittels externer Energiequellen gemäß Beispiel 16 Punkt 2 (Fig. 9) bzw. durch Änderung der Membranzusammensetzung gemäß Beispiel 16 Punkt 1 (Fig. 18) gegeben.

### Beispiel 6 Allgemeine Hormon- und Stoffwechselstörungen

Über eine dreidimensionale videomikroskopische Analyse von Nebennierenmarkszellen gemäß Beispiel 1 Punkt 2a, b wurde ein organisiertes TNT-basiertes Netzwerk zwischen den Zellen nachgewiesen, über welches ein Syncytium ausgebildet wird (Fig. 2e, f, Fig. 3n-q2, Fig. 4a-c) und Organellen (Fig. 4d-h, Fig. 14) transferiert werden. Weiterhin werden über TNTs zytoplasmatische Komponenten zwischen Zellen ausgetauscht. Dies wurde nachgewiesen, indem eine Population von neuroendokrinen PC12-Zellen (gemäß Beispiel 15 Punkt 7) mit CellTracker™ (Molecular Probes, "blue" C-2110) gefärbt (Population 2) und mit einer weiteren Population PC12-Zellen, die mit EGFP-Aktin (Fischer, M., Kaech, S., Knutti, D. & Matus, A. Rapid Actin-Based Plasticity in Dendritic Spines. Neuron (1998) 20: 847-854) transfiziert wurde (Population 1), gemischt und ausplattiert wurde. Die gemischten Zellkulturen wurden 24-48 Stunden später fixiert und immunzytochemisch mit einem polyklonalen Anti-GFP Antikörper (Molecular Probes A-6455) und Phalloidin-FITC gefärbt (gemäß Beispiel 15). Die Zellen wurden mittels dreidimensionaler Fluoreszenzmikroskopie und anschließender Dekonvolution der aufgenommenen Bilddaten analysiert (gemäß Beipiel 15 Punkt 2). EGFP-Aktin wurde selektiv in über TNT verbundenen Zellen von Population 2 nachgewiesen (Fig. 3j-m2), d. h. selektiv zwischen den TNT-verbundenen Zellen transferiert. Deshalb können TNTs eine elektrische oder auch chemische Zellkopplung ermöglichen und dadurch die synchronisierte Exozytose von Botenstoffe erwirken. Bislang konnten keine elektrischen oder sonstigen Verbindungen zwischen PC12-Zellen gefunden werden, was mit dem Fehlen von Connexinen, speziellen Kontaktproteinen, in diesen Zellen konsistent ist. Obwohl der erregende Nervus Splachnicus nur teilweise synaptische Kontakte mit den Nebennierenmarkszellen ausbildet, erfolgt eine synchronisierte Zellantwort des Nebennierenmarks. Dieses Prinzip von Zellkopplung über TNTs kann auch für andere Körper- oder Drüsengewebe Gültigkeit haben. Die fehlende Synchronisation der Nebennierenzellen nach dem Abreißen der TNTs z. B. durch Licht (Fig. 9) könnte z. B. eine nicht den physiologischen Bedürfnissen angepasste Sekretion von Signalstoffen zur Folge haben und damit kausal für viele Hormon- und Stoffwechselkrankheiten verantwortlich sein. Diese Stoffwechselstörungen können gemäß den unter Beispiel 3 angegebenen Verfahren wie z. B. der Abschirmung von externen Energieformen und/oder der Beeinflussung endogener Faktoren wie z. B. die Veränderung der Zellmembranzusammensetzung (gemäß Beispiel 16 Punkt 1) über Applikation spezfischer Pharmaka behandelt werden. Auch ist eine Therapie durch gezielte Stimulation der TNT-Bildung, z. B. über die Expression viraler Proteine wie z. B. des VSV G-ECFP (Fig. 17 d) denkbar.

### Beispiel 7 Mittel zur Verbesserung oder Schaffung neuer gentherapeutischer Verfahren

Ein zentraler Punkt für die Gentherapie ist die gezielte Verteilung reaktiver Komponenten in Gewebsverbänden. Dies wird z. B. mit der Kopplung solcher Komponenten an das virale VP22 Protein verfolgt, welches effizient nach einem bisher unbekannten Mechanismus von Zelle zu Zelle transferiert wird. Hierzu wurde der Transfer mechanismus im Hinblick auf eine Beteiligung von TNTs untersucht. 24 Stunden nach Transfektion von PC12-Zellen mit VP22-GFP wurden die Zellen mittels Videofluoreszenzmikroskopie analysiert (gemäß Beispiel 15). Es wurden stark fluoreszierende TNT-Verbindungen zwischen den Zellen gefunden (Fig. 17a-c). Innerhalb dieser Verbindungen waren fluoreszierende, vesikuläre Strukturen sichtbar, die im Verlauf der Beobachtung ihre Position veränderten. Diese Befunde dokumentieren einen TNT-abhängigen Transfer des viralen Proteins zwischen den Zellen. Wegen ihrer Eigenschaft, den Transport spezieller therapeutisch bedeutsamer Moleküle wie z.B. VP22 zu ermöglichen, stellen TNTs und ihre Beeinflussung wichtige Ansatzstellen dar, um gentherapeutische Verfahren zu verbessern oder sogar neue Verfahren zu schaffen. Zum Beispiel kann durch Stimulation der TNT-Bildung durch z. B. Expression viraler Proteine wie des VSVG-Proteins (Fig. 17d, gemäß Beispiel 8) bzw. der Beeinflussung von TNTs (gemäß Beispiel 16) Einfluss auf den interzellulären Transport von Therapeutika genommen werden. Die Möglichkeit, die dazu notwendigen Energien (z. B. Schall, elektromagnetische Felder, Licht) oder Pharmaka lokal zu applizieren, stellt eine lokale Steuerung der Therapeutika in Aussicht. Durch gezieltes Einbringen von therapeutischen Wirkstoffen, die mit den nötigen Signalen für einen Transport durch TNTs ausgestattet sind, lässt sich eine gezielte und effiziente Verbreitung im Gewebe gemäß Beispiel 1 Punkt 2 erreichen.

### Beispiel 8 Mittel zur Therapie von Infektionen mit Pathogenen

Neben dem Transfer von viralem VP22 gemäß Beispiel 7 wurde durch eine fluoreszenzmikroskopische Analyse von neuroendokrinen PC12-Zellen 48 Stunden nach der Transfektion (gemäß Beispiel 15) der interzelluläre Transfer eines weiteren viralen Fusionsproteins, VSVG-ECFP (Rustom, A., Bajohrs, M., Kaether, C., Keller, P., Toomre, D., Corbeil, D., Gerdes, H.-H. : Selective delivery of secretory cargo in Golgi-derived carriers of non-epithelial cells, in Traffic 2002, 3: 279-288), nachgewiesen (Fig. 17d). Darüber hinaus wurde in VSVG exprimierenden Zellen ein stimulierender Effekt auf die TNT-Bildung beobachtet. Es ist davon auszugehen, dass auch andere pathogene Proteine bzw. Pathogene wie die hier untersuchten Modellviren sich über TNTs im Gewebe ausbreiten. Damit stellen TNTs ideale Angriffspunkte für neue Therapien dar, die Verbreitung von Pathogenen im Gewebe zu unterbinden. Gezielte Zerstörung der TNTs durch lokale Applikation der dazu notwendigen Energieformen oder Pharmaka gemäß Beispiel 1 und Beispiel 16 kann die Verbreitung von Pathogenen damit vermindern oder gar unterbinden. Durch Beeinflussung der Membranzusammensetzung wie z. B. durch Pharmaka gemäß Beispiel 16 Punkt 1, die z. B. den Cholesteringehalt der Zellmembranen und damit die Stabilität der TNTs verändern (Fig. 18), oder durch gezielte Ernährungsumstellungen, lassen sich Präventivmaßnahmen zur Verhinderung viraler und bakterieller Infektionen finden.

### Beispiel 9 Subtile physiologische und psychologische Effekte

Die ultrasensiblen TNTs könnten auf Grund ihrer extremen Sensibilität für die Perzeption von externen Energieformen wie z. B. Infraschall, Ultraschall, Licht oder Magnetfelder bzw. auch interner Reize verantwortlich sein. Diese Annahme wird gestützt durch die Beobachtung, dass z. B. durch die relativ energiearme Anregung mit Licht der Wellenlänge 565 nm während den mikroskopischen Analysen TNTs zu mikroskopisch auflösbaren Schwingungen angeregt wurden und in vielen Fällen abrissen (Fig. 9). Solche Vibrationen bzw. das Abreißen von TNTs können vielfältige zelluläre Reaktionen auslösen. Im Falle der Reizung von Nebennierenzellen kann dies zu einer Erhöhung der neuroendokrinen Sekretion führen, und in der Folge z. B. zu Bluthochdruck. Dies wird gestützt durch die Beobachtung, dass die Neuropeptide und Hormone speichernden sekretorischen Granula gehäuft in den Kontaktzonen der durch TNTs verbundenen Zellen lokalisiert sind (Fig. 20). Die z. B. aus der Nebenniere in die Blutbahn freigesetzten Stoffe enthalten Komponenten, die sowohl auf die physiolögischen Voränge anderer Organe und Gewebstypen wirken als auch die synaptische Plastizität neuronaler Netzwerke im Zentalnervensystem verändern. Denkbar ist, dass dies subtile physiologische und psychologische Effekte zur Folge haben kann. Beispiele für solche Effekte schließen allgemeine Beschwerden wie Unwohlsein, Unruhe, Schlafstörungen und Nervosität bis hin zu schizophrenen Verhaltensweisen als auch unbewusste Reaktionen auf Wasseradern, Handystrahlung, Hochspannungsleitungen, Industrieanlagen bis hin zu parapsychologischen Effekten ein. Therapieansätze sind durch Beeinflussung der TNT-Verbindungen bzw. deren Bildung gemäß den Beispielen 2, 3, 6 und 8 (Abschirmen bzw. Elimieren externer Energieformen, Beeinflussung der Lipidzusammensetzung der Zellmembran, Induktion der Bildung von TNTs) gegeben.

### Beispiel 10 Gewebezüchtung ("Tissue engineering")

Über eine dreidimensionale videomikroskopische Analyse von Nebennierenmarkszellen gemäß Beispiel 1 Punkt 2a, b wurde die Ausbildung eines organisierten TNT-basiertes Netzwerk zwischen den Zellen nachgewiesen, über welches ein Syncytium entsteht (Fig. 2e, f, Fig. 3n-q2, Fig. 4a-c) und Organellen unidirektional (Fig. 4d-h, Fig. 14) tranferiert werden können. TNTs wurden gemäß Beispiel 16 in allen bisher untersuchten Zellkultursystemen wie PC12-Zellen, HEK-Zellen (Fig. 16a) sowie Primärkulturen der Medulla (Fig. 16b) und des Hippocampus (Fig. 16c) gefunden. Mit einer speziellen Anfärbemethode wurden zudem sehr lange TNTs gefunden. Dazu wurden PC12-Zellen in einer LabTek™-Zellkulturschale durch vorsichtige Zugabe (lokale Applikation) von Dil (Molecular Probes, Oregon) zum Zellkulturmedium gefärbt. Während der Färbung und der simultan stattfindenden mikroskopischen Analyse (gemäß Beispiel 15) wurden, soweit möglich, destruktive Energieformen (Erschütterungen, Bewegungen des Mediums, zu starke Lichteinstrahlung usw.) vermieden. Die Analysen zeigten TNTs, die einzelne Zellen über Distanzen von mehr als einem Millimeter verknüpfen (Fig. 19). Die Bildung von TNTs stellt damit eine wesentliche Komponente für Gewebsbildung in sich entwickelnden oder adulten Organismen dar. Es wurde gezeigt, dass TNTs nicht aus der Zellteilung über eine unvollständige Zytokinese hervorgehen (Fig. 12), sondern *de novo* gebildet werden (Fig. 2a-d). Hierzu wurden PC12-Zellen nach 14-stündiger Behandlung mit 2,5 mM Thymidin im Zellkulturmedium, welches die die Zellen in der G1/S-Phase des Zellzyklus blockiert und eine Teilung verhindert, oder unter Kontrollbedingungen, d. h. ohne Gegenwart von Thymidin, passagiert und eine Stunde später (gemäß dem Beispiel 15) nach Färbung mit WGA, 20 Minuten Fixierung in 4% Paraformaldehydl 4% Sucrose, 3 Minuten Behandlung mit 0,2% Triton™ X-100 und Kemfärbung mit Dapi. auf die Bildung von TNTs hin analysiert (Fig. 12). Diese Ergebnisse implizieren, dass die Gewebszüchtung *in vitro* stark von der Ausbildung und/ oder dem Erhalt von TNTs abhängt. Das trifft nicht nur für stark proliferierende Zellen zu, sondern auch für langsam wachsende oder postmitotische Zellkulturen. Dies widerum legt nahe, dass eine Steuerung von *in vitro* Gewebskulturen durch Modulation der TNT-Verbindungen erzielt werden kann. Diese Modulation basiert auf ihren charakteristischen Eigenschaften, auf die gemäß Beispiel 16 durch externe Energieformen wie z. B. Licht (Fig. 9) bzw. Änderung der Lipidzusammensetzung wie z. B. Cholesterinentzug (Fig. 18) gezielt Einfluss genommen werden kann.

### Beispiel 11 Computergestützte neuronale Netzwerke

Gemäß Beispiel 10 wurde die Ausbildung eines organisierten TNT-basierten Netzwerks zwischen den Zellen nachgewiesen, über welches ein Syncytium entsteht (Fig. 2e, f, Fig. 3n-q2, Fig. 4a-c) und Information unidirektional (Fig. 4d-h, Fig. 14) zwischen einzelnen Zellen ausgetauscht wird. Gemäß Beispiel 16 wurden TNTs auch in neuronalen Primärkulturen gefunden (Fig. 16c). Deshalb ist davon auszugehen, dass TNTs eine entscheidende Komponente der Informationsverarbeitung neuronaler Systeme darstellen. Computergestützte neuronale Netzwerke basieren auf der Simulation solcher neuronaler Netzwerke des zentralen Nervensystems. Das Potential solcher Netzwerksimulierungen wird für zukünftige Technologien sehr hoch eingeschätzt, obwohl die zur Zeit realisierten Systeme im Vergleich zum zentralen Nervensystem noch stark in ihrer Funktion limitiert sind. Über das gefundene Verschaltungsmuster von TNT-verbundenen Zellen in Kombination mit dem unidirektionalen Transfer von Information besteht die Möglichkeit, die gegenwärtigen computergestützten Netzwerke durch Berücksichtigung der auf TNTs basierenden Verbindungen und deren charakteristischen Eigenschaften maßgeblich zu verbessern oder neue Systeme zu entwickeln.

### Beispiel 12 Färbeverfahren und Charakterisierung von TNTs

Unsere Untersuchungen ergaben, dass TNTs *in vivo* außer mit WGA (Fig. 1 a-d) auch durch Transfektion mit Plasmiden, welche für GFP-gekoppeltes Vesicular Stomatitis Virus G-Protein (virales Transmembranprotein) (Fig. 17d), für EGFP-Aktin (Abb. 3j-m2) oder für farnesyliertes EGFP (Fig. 3n-q2, Fig. 4a-c), einem sehr spezifischen Plasmamembranmarker (Fig. 8) kodieren, sichtbar gemacht werden können. Weiterhin konnten ,', TNTs *in vitro* nach vorsichtiger Fixierung durch die Anfärbung von Aktin mit Phalloidin visualisiert werden (Fig. 1e). Es wurde gefunden, dass im Unterschied zu Axonen und vielen Filopodien TNTs ausschließlich Aktin und keine Mikrotubuli enthalten (Fig. 1e, Fig. 6). Für diesen Nachweis wurden PC12-Zellen 24 Stunden nach dem Ausplattieren mit Glutaraldehyd fixiert (gemäß Fig. 1f), immuncytochemisch mit Antikörpern gegen Alpha-Tubulin (Fig. 6a, b, c1, d1) und mit Phalloidin-FITC (Fig. 6c2. d2) gefärbt und anschießend mittels Fluoreszenzmikroskopie analysiert (gemäß Beispiel 15). Mikrotubulistränge sind in den Filopodien der Zellen zu sehen (Fig. 6a, b) und in gebündelter Form in der "Midbody"-Region (Fig. 6c1) von Zellen, die während der Zytokinese fixiert wurden und deshalb den kontraktilen Aktinring zwischen den Zellen (Fig. 6c2) aufweisen. Bei TNT-verbundenen Zellen zeigt sich, dass in dem TNT keine Mikrotubuli (Fig. 6d1), dagegen jedoch filamentöses Aktin (Fig. 6d2) nachgewiesen werden kann. Mit Ausnahme von Aktin (Fig. 3j-m2) konnte der TNT-vermittelte interzelluläre Transfer löslicher, nicht polymerer cytoplasmatischer Stoffe bislang nicht gezeigt werden. Für niedermolekulare Farbstoffe wie z. B. Bodipy™ oder Calcein AM sind TNTs vermutlich nicht durchlässig (vergleiche Fig. 13). Diese Beobachtung würde widerum auch erklären, warum TNTs während Mikroinjektionsstudien von diesen oder ähnlichen Farbstoffen nicht sichtbar waren bzw. kein Transfer dieser Farbstoffe beobachtet werden konnte. Der stark eingeschränkte TNT-vermittelte Transfer kleiner Moleküle wie Calcein und cytoplamatisch exprimierter Proteine wie z. B. EYFP (Fig. 7) und vermutlich auch Ionen ist in Übereinstimmung mit der bislang nicht nachweisbaren elektrischen Kopplung von TNT- verbundener Zellen ("patch-clamp"-Verfahren). Dagegen wurde der TNT-vermittelte Transport von kleinen Organellen/Vesikeln, Aktin und Plasmamembrankomponenten wie z. B. famesyliertem EGFP von Zelle zu Zelle beobachtet (Fig. 3, 4). Im Gegensatz zu Filopodien und Axonen sind TNTs gegenüber Trypsinbehandlungen nicht sensitiv (Fig. 10). Dies wurde gezeigt durch zeitversetzte Fluoreszenz- und Hellfeld-Videomikroskopieaufnahmen von TNTs zwischen PC12-Zellen, die in Zellmedium einer Behandlung mit 1,25% (Fig. 10a1, a2) bzw. 2,5% (Fig. 10b1, b2) Trypsin/EDTA unterzogen wurden. Die andauernde Trypsin/EDTA-Behandlung führt zur Ablösung der Zellen vom Substrat sichtbar durch die zunehmende Aufrundung der Zellen und schließlich zu deren Ablösung (Fig. 10). Die TNTs bleiben über den beobachteten Zeitraum intakt (Fig. 10).

### Beispiel 13 Mikroskopische Analyse

PC12-Zellen (Heuman R. et al., Relationship between NFG-mediated volume increase and "priming effect" in fast and slow reacting clones of PC12 pheochromacytoma cells, Exp. Cell Res. 145, 179-190 (1983)) wurden in LabTek™-Zellkulturgefäßen bei optimaler. Dichte kultiviert (siehe Rudolf R. et al., Dynamics of immature secretory granules: role of cytoskeletal elements during transport, cortical restriction and F-actindependent tethering. Mol. Biol. Cell, 12, 1353-1365 (2000)). Das Zellkulturmedium enthielt DMEM, 10% Pferdeserum (Gibco), 5% fötales Kälberserum (Gibco), 4 Millimolar Glutamin. Ein Kulturgefäß wurde mit z. B. pharmakologischen Substanzen oder anderen Einflüssen (z. B. externe Energieformen) behandelt. Ein Kontrollgefäß wurde unter standardisierten Bedingungen gehalten. Nach dem Experiment wurden Kontroll- und Versuchszellen mit WGA gefärbt (siehe nachstehendes Beispiel) und auf TNTs hin analysiert. Dazu wurde eine automatisierte dreidimensionale Videomikroskopie lebender Zellen durchgeführt. Hierzu wurden in frei definierbaren Bereichen des Zellkulturgefäßes dreidimensionale Bildstapel der Zellen aufgenommen. Das Mikroskopiesystem umfasste hierzu einen programmierbaren Mikroskop-Tisch, eine Autofokuseinrichtung, einen Z-Stepper sowie geeignete Steuerungssoftware. Im Gegensatz zu zweidimensionalen Analysen konnten mit Hilfe dieser dreidimensionalen Bildstapel die Anzahl und das Erscheinungsbild der TNTs präzise bestimmt werden. Durch die Applikation von GFP-Fusionsproteinen oder anderer vitaler Fluoreszenzfärbungen zur selektiven Markierung bestimmter über TNTs ausgetauschter membranöser Strukturen konnte eine quantitative Analyse dieses Austausches mittels der beschriebenen automatisierten dreidimensionalen Mikroskopie lebender Zellen erfolgen. Diese Verfahren erfolgten mit Einfarben- oder Mehrfarbenanalyse. Eine Analyse von TNTs in Zellkulturen war auch nach vorsichtige Fixierung und anschließender Färbung mit z. B. Phalloidin möglich, nach unserem Dafürhalten jedoch weniger verlässlich, da die harsche Fixiermethode partiell zu einer Zerstörung von TNTs führte und bestehende Unterschiede zwischen Versuchs- und Kontrollzellen veränderte.

### Beispiel 14 FACS-Analyse

Es wurden zwei PC12-Zellenkulturen separat mit Dil oder DiO™ gefärbt. Vibrant™ Dil sowie DiO (Molecular Probes, Eugene, Oregon) wurden nach Angaben der Hersteller direkt für die Markierung der Zellen in Suspension eingesetzt. Dazu wurden die PC12-Zellen in der 15 cm Zellkulturschale 2 mal mit 1 x PBS gewaschen und 2 bis 5 Minuten mit 1 ml Trypsin bei 37° C inkubiert. Es wurde 5 ml PC12- Zellkulturmedium zupipettiert und die Zellen 5 Minuten bei 400 UpM/4°C abzentrifugiert. Der Überstand wurde abgesaugt und das Zellpellet in 1 ml 1x PBS resuspendiert. Es wurden 5 Mikroliter Vibrant™ Dil bzw. DiO "Cell Labelling Solutions" (Molecular Probes, V-22889) hinzugemischt und die Proben 20 Minuten im Inkubator bei 37° und 10% CO₂ bei gelegentlichem Rütteln inkubiert. Dann wurden die Zellen erneut abzentrifugiert (5. Minuten bei 400 UpM/4°C). Der Überstand wurde verworfen und das Zellpellet in 3 Milliliter Kulturmedium, erwämt auf 37°C, resuspendiert. Die letzten beiden Schritte wurden zweimal wiederholt. Zum Schluss wurden die Dil bzw. DiO gefärbten Zellpellets in jeweils 1 Milliliter Zellkulturmedium aufgenommen, gemischt, auf ca. 40 Milliliter mit Zellkulturmedium verdünnt und auf Zellkulturschalen ausplattiert. Die Analyse unter dem Fluoreszenzmikroskop zeigte, dass sowohl Dil als auch DiO nahezu vollständig in vermutlich endozytierten membranösen Strukturen erscheinen und kaum an der Zellmembran nachweisbar sind (Fig. 4d-h). Zu frühen Zeiten nach dem Ausplattieren der Mischkultur lagen Zellen mit ausschließlich grünen oder roten Fluoreszenzsignalen vor (Fig. 4f). Bereits 2 Stunden später fanden sich TNT-verbundene Zellen, die sowohl grüne als auch rote Fluoreszenzsignale enthielten (Fig. 4g1). Mit der Zeit nahm sowohl die Anzahl dieser Zellen als auch die Anzahl der ausgetauschten fluoreszenten Strukturen zu (Fig. 4h). Nach 24 bis 48 Stunden wurden in den Zellen überwiegend gelbe und wenige grün und rot fluoreszierende Strukturen beobachtet (Fig. 4h), was auf die Fusion der ausgetauschten grünen und roten membranösen Strukturen hinweist. Der über TNTs erfolgende unidirektionale Austausch von Membranvesikeln konnte so einfach mittels "Fluorescence Activated Cell Sorting" (FACS)-Sortierung quantifiziert werden, was ein verblüffend einfaches, sehr effizientes und quantitatives Verfahren ist und somit auch geeignet für pharmakologische Reihentests zur Untersuchung einer Beeinflussung von TNTs und der darüber realisierten Zell-Zell-Kommunikation.

### Beispiel 15 Mikroskopierverfahren

### 1. Zellpassage von PC12-Zellen

Auf 15 Zentimeter Zellkulturschalen (Nalge Nunc International) kultivierte Zellen wurden 2 mal mit 1 x PBS gewaschen, dann 1 Milliliter Trypsin (Trypsin/EDTA-Lösung, Gibco-BRL) zugegeben und die Zellen etwa 5 Minuten bei 37°C (10% CO₂) inkubiert. Die Zellen wurden in 5 Milliliter Zellkulturmedium (siehe Material) aufgenommen und 5 Minuten bei 400 UpM (4°C) abzentrifugiert. Das Zellpellet wurde in 2 Milliliter Zellkultur medium resuspendiert und aufgenommen, zur Zellenvereinzelung ca. 80 bis 100 mal mit einer verengten Glaspipette auf- und abpipettiert und im gewünschtem Volumen Zellkulturmedium verdünnt. Die Zellen wurden dann auf PLL (Poly-L-Lysin) beschichteten LabTek™-Zellkulturschalen (Chambered Coverglas 4 weil, Nalge Nunc International) in gewünschter Dichte ausplattiert.

### 2. Visualisierung von TNTs am Mikroskop:

Das mikroskopische System bestand aus einem Olympus IX 70 Mikroskop, einem Pl Piezo-z-Stepper (E-662, Physik Instrumente GmbH & Co.), einer Wärmebox (Life Imaging Services, Olten, Schweiz), einem Polychrome II Monochromator (T.I.L.L. Photonics GmbH, Martinsried, Deutschland), Dapi / FITC/ TRITC F61-020 Fluoreszenzfiltern (AHF Analysentechnik AG, Tübingen, Deutschland), und Steuerungssoftware TiIIVsion™ von T.I.L.L. Photonics GmbH, Martinsried, München. Die Ein- oder Mehrfarbenfluoreszenzvideomikroskopie erfolgte nach Standardmethoden mittels des beschriebenen Systems. Das gleiche gilt für dreidimensionale Fluoreszenz- oder Hellfeldmikroskopie. Um TNTs sehen zu können, musste erst die richtige mikroskopische Ebene überhalb des Bodens des Zellkulturgefäßes gefunden werden. Oft verliefen die TNTs nicht horizontal zum Zellkulturgefäß, sondern besaßen hierzu einen gewissen Neigungswinkel, wodurch die TNTs nicht komplett in einer mikroskopischen Ebene aufgelöst werden konnten. Das TNT konnte dann nur durch Durchfokussieren oder dreidimensionalen Studien komplett dargestellt werden. Dazu wurden in der Regel beginnend von der Zellbasis 40 Aufnahmen aufeinanderfolgender z-Schnittebenen mittels Z-Stepper durch die Zelle erstellt. Diese Bildstapel wurden zum Teil mit der Dekonvolutionserweiterung der TILLvisION-Software bearbeitet und mit einer geeigneten Software zur dreidimensionalen Analyse (durch Voxblast™, TILLvisION oder IPLab Software v3.2.2. von Scanalytics Inc., Fairfax, Virgina) dreidimensional rekonstruiert und analysiert. Die Konfokale Mikroskopie erfolgte mit einem Leica SP2-Konfokal-Mikroskop, ausgerüstet mit einem 100x HCX PL APO 100x/1.40 NA-C5lobjektiv (Leica Microsysteme Vertrieb GmbH, Bensheim, Deutschland). Die beiden verwendeten Abbildungssysteme waren ausgerüstet mit einer 37°C-Wärmeregelungsvorrichtung (Live Imaging Services, Olten, Schweiz).

### 3. Weizenkeim-Agglutinin-Färbung von TNTs

Die auf der LabTek™-Zellkulturschale gewachsenen Zellen wurden auf dem auf 37°C vorgeheizten Mikroskop in 450 Mikroliter Medium direkt mit ca. 1 Mikroliter "WGA Alexa Fluor™ 594" (1 Milligramm /Milliliter Lösung) von Molecular Probes (Eugene, Or. W-11262) versetzt. Nach ca. 2 bis 5 Minuten waren dann die Plasmamembranen der Zellen gefärbt und der Hintergrund im Medium reduziert.

### 4. Immuncytochemische Verfahren

PC12-Zellen wurden 24 Stunden nach Zellpassage vorsichtig 2 mal in 1 x PBS gewaschen und vorsichtig 20 Minuten in 4% PFA/ 4% Sucrose fixiert. Dann wurden die Zellen sorgsam 10 Minuten in 50 Millimolar NH₄Cl inkubiert. Die Antikörperfärbung erfolgte nach Standardmethoden, dabei wurde aber auf eine vorsichtige Behandlung der Proben geachtet. Bei den Färbungen der Kerne mit 4,6-Diamino-2-phenylindol-dihydrochlorid (Dapi) (Molecular Probes, Verdünnung 1:500) und den Aktinfärbungen mit Phalloidin wurden die Farbstoffe so eingesetzt, als wären sie Antikörper. F-Aktin wurde mit Phalloidin-TRITC/FITC-Konjugat (Sigma Chemical Co., 250 Nanomolar Endkonzentration) fluoreszenzmarkiert; siehe auch Rudolf R. et al, Dynamics of immature secretory granules: role of cytoskeletal elements during trn*sport, cortical restriction and F-actin dependent tethering, Mol. Biol. Cell, 12. 1353-1365 (2001). Die indirekte Immunfluoreszenzmarkierung erfolgte gleichfalls wie bei Rudolf R. et al beschrieben. Weiter eingesetzte Primärantikörper waren: polyklonaler Anti-GFP-Antikörper (Molecular Probes A-6455); monoklonaler Anti-Synaptophysin-Antikörper Sy-38 (Chemicon, Temecula, CA, Verdünnung 1:100); polyklonales Anti-Myosin-Va-Aniserum-Dil2 (Verdünnung 1:300) von Wu X. et al., Myosin V associates with melanosomes in mouse melanocytes: evidence that myosin V is an organelle motor., J. Cell Sci. 110. 847-859 (1997); monoklonaler Antialpha-Tubulin-Antikörper (Klon DM1A, Sigmal Chemical Co., Verdünnung 1:500). Als Sekundärantikörper wurden eingesetzt: Ziege-Anti-Maus-Lissamin (Verdünnung 1:500), Ziege-Anti-Kaninchen-TRITC (Verdünnung 1:200) und Ziege-Anti-Kaninchen-Cy5 (Verdünnung 1:500), alle von Jackson Immuno Research Labs, Inc. (West Grove, Pennsylvania).

### 5. Lysotracker™-Färbung

Vor dem Transfer der PC12-Zellen zum Mikroskop wurde vorsichtig das Zellkulturmedium gegen Zellkulturmedium mit 60 Nanomolar Lysotracker™ Green DND-26 ((Molecular Probes, Eugene, Or, L-7526) getauscht. Die gefärbten Zellen konnten direkt mit Hilfe der Fluoreszenzmikroskopie analysiert werden.

### 6. Transfektion von PC12-Zellen

Die Transfektion von cDNAs in PC12-Zellen erfolgte wie von uns in der Literatur beschrieben [Kaether C. et al., Targeting of green fluorescent protein to neuroendocrine secretory granules: a new tool for real time studies of regulated protein secretion. Eur. J. Cell Biol., 74, 133-142 (1997)].

### 7. CellTracker™-Färbung

Die Färbung wurde nach Angaben des Herstellers mit adhärenten Zellen durchgeführt, d. h. 15 Minuten Inkubation der Zellen in Medium, welches ..enthielt, gefolgt von 30 Minuten Inkubation in Medium ohne Farbstoff.

### Beispiel 16 Verfahren zum Testen der Beeinflussung von TNTs

Außer in PC12 -Zellen wurden TNTs auch in nicht-neuroendokrinen HEK-293-Zellen (ATCC CRL 1573 - menschliche embryonale Nierenzellen) zwei Tage nach Zellpassage (Fig. 16a), Medulla-Primärkulturen der Ratte (Fig. 16b, zur Isolierung vereinzelter Zellen wurde das Nebennierenmark von P10-Ratten einer Kollagenase- (0.1%) und wiederholten Trypsin-Behandlungen (0.125%) unterzogen; die vereinzelten Zellen wurden anschließend auf Polyornithin/Laminin beschichtete LabTek™ Zellkulturschalen ausplattiert und 4 Tage kultiviert) sowie hippocampalen Primärkulturen (Fig. 16c, präpariert nach Standardmethoden (Banker, G. Goslin, K. in Culturing Nerve Cells, eds. Banker G & Goslin K, MIT Press, Cambridge MA, 1991)) nach Färbung mit WGA durch hochauflösende, dreidimensionale Videomikroskopieaufnahmen nachgewiesen (gemäß Beispiel 15). Die Morphologie und die Anzahl der in diesen Kulturen gebildeten TNTs lässt sich mittels einer dreidimensionalen mikroskopischen Analyse gemäß Beispiel 13 bestimmen. Deshalb liefern diese oder ähnliche Zellkulturen in Kombination mit den in Beispiel 13 beschriebenen Analyseverfahren Screening Systeme, um die Beeinflussung von TNTs durch z. B. Zytopharmaka oder externe Energieformen zu testen.

### 1. Screening von Zytopharmaka zur Beeinflussung von TNTs

Um den Einfluss von z. B. Cholesterin auf die Stabilität von TNTs zu untersuchen, wurde das Zellkulturmedium von PC12-Zellen zwei Stunden nach Passage gegen DMEM mit 5, 8 oder 10 Millimolar Saccharose (Kontrollen) oder DMEM mit 5, 8 oder 10 mM Methyl-β-cyclodextrin ausgetauscht und die Zellen für eine halbe Stunde bei 37° C und 10% CO₂ inkubiert. Nach Zugabe von WGA wurde eine dreidimensionale Analyse von 10 zufällig ausgewählten mikroskopischen Feldern durchgeführt (Olympus IX70, 100x Objektiv, TillvisiON System, Piezo-z-stepper, je 40 Schnitte) und die Anzahl der TNTs bestimmt. Fig. 18a1 und Fig. 18b1 zeigen für die 5 Millimolar Methyl-β-cyclodextrin-Bedingung representative Bildebenen der dreidimensionalen Analyse, in denen TNTs mit Pfeilspitzen markiert sind. Die Quantifizierung ergab eine starke Abnahme der TNT-Anzahl mit steigender Methyl-β-cyclodextrin Konzentration: ca. 40% Abnahme bei 5 Millimolar Methyl-β-cyclodextrin; ca. 93% Abnahme bei 8 Millimolar Methyl-β-cyclodextrin; 100% bei 10 Millimolar Methyl-β-cyclodextrin (Fig. 18c1). Um den Effekt von Methyl-βcyclodextrin zur Senkung des zellulären Cholesteringehalts nachzuweisen, wurde eine Filipin-Färbung (Sigma, F-9765) der Zellen durchgeführt. Dazu wurde das Methyl-βcyclodextrin- bzw. Saccharose-haltige DMEM der analysierten Zellen durch DMEM mit 20 Mikrogramm pro Milliliter Filipin ausgetauscht und 15 Minuten später die Intensität der Fluoreszenzfärbung *in vivo* mit einem FITC/TRITC/DAPI-Filtersatz (Chroma, Brattleboro) analysiert. Im Detail wurden nach Anregung mit 400 Nanometer vier zufällig ausgewählte mikroskopische Felder aufgenommen und die mittleren Graustufenwerte bestimmt. Die Auswertung nach Behandlung mit 5 Millimolar Methyl-β-cyclodextrin ergab eine Reduktion des zellulären Cholesteringehalts um 20% (Fig. 18c2). Gemäß diesem Beispiel können andere Zytopharmaka bzgl. eines Einflusses auf TNTs und der davon abhängigen zellulären Kommunikation getestet werden.

### 2. Screening von externen wellenförmigen Energieformen

In der PC12-Zelllinie wurden TNTs nach Färbung mit WGA durch hochauflösende, dreimensionale Videomikroskopieaufnahme circa 24 Stunden nach Zellpassage nachgewiesen (Fig. 1a-d). Der Einfluss von externen Energieformen wie z. B. sichtbares Licht eines Mikroskopischen Systems gemäß Beispiel 15 Punkt 2 mit einer Wellenlänge von 565 Nanometer wurde an diesen Zellen getestet. Über einen Beobachtungszeitraum von ca. 70 Sekunden, in dem die Verbindung (Fig. 9a) von dem einfallenden Licht der Wellenlänge 565 nm zunächst in Schwingung versetzt wird (Fig. 9b), reißt (Fig. 9c) und sich innerhalb Sekunden an seinem losen Ende wie ein gerissenes Gummiband aufwickelt (Fig. 9d). Mit diesem Modellsystem können auch die Einflüsse anderer wellenför miger Energieformen auf TNTs analysiert werden und die für z. B. Therapieansätze effektivsten und selektivsten Frequenzen und Intensitäten der verwendeten Energieformen zu ermitteln.

### Beispiel 17 Prinzip von Membrankontinuität zwischen Tierzellen

TNTs bilden ein Membrankontinuum zwischen den verbundenen Zellen aus. Ferner stellen TNTs vermutlich ein neues und generelles zelluläres Prinzip dar. Diese Annahmen werden unter anderem gestützt durch Experimente die den Transfer von Plasmamembrankomponenten (Fig. 3n-q2, Fig. 4a-c) und Aktin (Fig. 3j-m2) sowie den direkten Transfer von Dil-gefärbten Organellen (Fig. 4d, e, Fig. 14) zwischen TNT-verbundenen Zellen zeigen. Ein vergleichbares Membrankontinuum wird in pflanzlichen Organismen durch *Plasmodesmata* zwischen den Zellen realisiert. Bei tierischen Organismen ist *de novo* Bildung eines Membrankontinuums zwischen Zellen nicht beschrieben. Die gegenwärtige Auffassung ist die, dass Tierzellen in der Regel Individuen darstellen. Das hier vorgeschlagene Modell TNT-vermittelter Membrankontinuität zwischen Tierzellen zusammen mit den gefundenen Transportvorgängen (Abb. 5) erlaubt damit prinzipiell den Austausch verschiedenster endogener oder exogener Komponenten zwischen den verbundenen Zellen. Wichtige, in Frage kommende Komponenten für einen solchen Austausch sind z.B. Morphogene oder Transkriptionsfaktoren die für die Entwicklung bzw. den Erhalt von Organismen essentielle Bedeutung haben. Über die in Beispiel 16 dargestellten Möglichkeiten, TNTs zu beeinftussen ergeben sich weitreichende Möglichkeiten gezielt und fein-regulierbar auf vielfältige biologische Mechanismen Einfluss zu nehmen.

## Patentansprüche

1. Verfahren zur Untersuchung von interzellulärer Kommunikation und interzellulärem Transport, wobei Zellen nach Vereinzelung auf Membrantuben hin untersucht werden, die F-Actin und Myosin enthalten, einen Durchmesser von 50 bis 400 Nanometer besitzen, bis zu 50 Mikrometer lang sind und die sich zwischen den Zellen spannen.

2. Verfahren nach Anspruch 1, wobei der Organellentransport zwischen den Zellen untersucht wird.

3. Verfahren nach Anspruch 2, umfassend die Schritte:
(i) Zusetzen von ein oder mehreren Stoffen zu einer ersten Anzahl Zellen, wobei der Stoff so gewählt ist, dass er innerhalb einer ersten Zeitdauer von den Zellen der ersten Anzahl endozytiert wird;
(ii) Mischen der ersten Anzahl Zellen nach Waschen mit einer zweiten Anzahl Zellen, so dass sich zwischen den Zellen der ersten und der zweiten Anzahl innerhalb einer zweiten Zeitdauer interzelluläre Membrantuben bilden, und
(iii) Bestimmen der Zahl der Zellen aus der zweiten Anzahl Zellen, die die ein oder mehrere endozytierte Substanzen enthalten.

4. Verfahren nach Anspruch 3, wobei in Schritt (i) die erste Anzahl Zellen mit einem ersten endozytlerbaren Stoff und die zweite Anzahl Zellen mit einem zweiten endozytierbaren Stoff versetzt wird, wobei erste und zweite Substanz verschieden sind.

5. Verfahren nach Anspruch 3 oder 4, wobei die endozytierbaren Substanzen gewählt sind aus ein oder mehreren Farbstoffen, Fluoreszenzfarbstoffen, Dil, DiO, radioaktiven Markersubstanzen, Lumineszenzfarbstoffen, fluoreszierenden oder lumineszierenden Proteinen und Peptiden, Proteinen oder Peptiden, die gekoppelt sind mit einer Markersubstanz gekoppelt sind.

6. Verfahren nach irgendeinem der Ansprüche 2 bis 5, wobei in Schritt (i) die Endozytose der Substanz in eine Organelle ersetzt ist durch eine konstitutive oder transiente Expression der Substanz in die Organelle.

7. Verfahren nach irgendeinem der Ansprüche 2 bis 6, wobei der Schritt (iii) durch FACS erfolgt.

8. Verfahren nach irgendeinen der Ansprüche 2 bis 7, wobei Schritt (ii) in Anwesenheit bzw. unter Einwirkung eines Profmittels erfolgt.

9. Verfahren nach Anspruch 8, wobei das Prüfmiftel eine chemische Verbindung oder eine mutmaßliche pharmazeutische Wirksubstanz ist.

10. Verfahren nach Anspruch 8, wobei das Prüfmittel ein Arzneimittel oder Therapeutikum ist.

11. Verfahren nach Anspruch 8, wobei das Prüfmittel eine physikalische Vorrichtung ist.

12. Verfahren nach Anspruch 8, wobei das Prüfmittel eine physikalisch therapeutische Vorrichtung ist.

13. Verfahrens nach Anspruch 1, wobei die Untersuchung mit einem mikroskopischen System erfolgt, welches die Beobachtung von verschiedenen mikroskopischen Ebenen in der Z-Achse erlaubt.

14. Verfahren nach Anspruch 13, wobei das mikroskopischen System ein Mikroskop, einen z-Stepper und eine zugehörige Steuerung umfasst.

15. Verwendung des Verfahrens nach einem der Ansprüche 1 bis 14 zur Reihenuntersuchung von mutmaßlichen Arzneimitteln und Wirkmitteln.

16. Verwendung des Verfahrens nach Anspruch 15 zur Reihenuntersuchung von mutmaßlichen Wirkstoffen und Wirkmitteln zur Behandlung von Tumoren, des Bluthochdruck, von viralen, bakteriellen oder parasitischen Infektionserkrankungen, Erkrankungen des Stoffwechsels, Erkrankungen des Nervensystems, der Psyche und des Geistes, des Cholesterinspiegels, sowie auf dem Giebiet der Geloebezüchtung.

17. Verwendung des Verfahrens nach Anspruch 15 zur Untersuchung von Wirkstoffen in der Gentherapie, zum Zelltargeting und in der Pharmakologie, und in der Gewebezüchtung.

## Claims

1. Method for the investigation of intercellular communication and intercellular transport, wherein after singularisation cells are investigated for membrane tubes which contain F-actin and myosin, have a diameter of 50 to 400 nm, are up to 50 micrometers long or in same cases longer, and which span between the cells.

2. Method according to claim 2, wherein the organelle transport between the cells is investigated.

3. Method according to claim 2, including the steps:
(i) addition of one or more substances to a first number of cells, wherein the substance is so selected that it is endocytosed by the cells of the first number within a first time period;
(ii) mixing of the first number of cells after washing with a second number of cells, so that within a second time period intercellular membrane tubes are formed between the cells of the first and second number, and
(iii) determination of the number of the cells of the second number of cells which contain the one or more endocytosed substances.

4. Method according to claim 3, wherein in step (i) the first number of cells are those with a first endocytable substance and the second number of cells are those with a second endocytable substance, wherein the first and second substances are different.

5. Method according to claim 3 or 4, wherein the endocytable substances are selected from one or more dyes, fluorescence dyes, Dil, DiO, radioactive marker substances, luminescence dyes, fluorescing or luminescing proteins and peptides, proteins or peptides which are coupled with a marker substance.

6. Method according to any of claims 2 to 5, wherein in step (i) the endocytosis of the substance is replaced in an organelle by means of a constitutive or transient expression of the substance in the organelle.

7. Method according to any of claims 2 to 6, wherein step (iii) is effected by means of FACS.

8. Method according to any of claims 2 to 7, wherein step (ii) is effected in the presence or under the effect of a test medium.

9. Method according to claim 8, wherein the test medium is a chemical compound or suspected pharmaceutically effective substance.

10. Method according to claim 8, wherein the test medium is a medicament or therapeutic substance.

11. Method according to claim 8, wherein the test medium is a physical device.

12. Method according to claim 8, wherein the test medium is a physical therapeutic device.

13. Method according to claim 1, wherein the investigation is effected with a microscope system which allows the observation of different microscopic planes in the z-axis.

14. Method according to claim 13, wherein the microscopic system includes a microscope, a Z-stepper and an associated controller.

15. Use of the method according to any of claims 1 to 14 for a screening of suspected pharmaceuticals and active ingredients.

16. Use of the method according to claim 15 for a screening of suspected active substances and active ingredients for the treatment of tumours, of high blood pressure, of viral, bacterial or parasitic infection diseases, disorders of the metabolism, disorders of the nervous system, the psyche or the mind, of the cholesterol level, and in the field of tissue culture.

17. Use of the method according to claim 15 for the investigation of effective substances in gene therapy, for cell targeting and in pharmacology, and in the field of tissue culture.

## Revendications

1. Procédé pour examiner une communication intercellulaire et un transport intercellulaire dans lequel les cellules sont examinées après désassortiment en vue de déterminer la présence de tubes membranaires contenant de l'actine-F et de la myosine, ayant un diamètre de 50 à 400 nanomètres, une longueur jusqu'à 50 micromètres et qui s'étendent entre les cellules.

2. Procédé selon la revendication 1 dans lequel le transport d'organelles entre les cellules est examiné.

3. Procédé selon la revendication 2 comprenant les étapes :
(i) ajouter un ou plusieurs matières à un premier groupe de cellules, la matière étant choisie de telle manière qu'il est endocyté pendant une première durée par les cellules du premier groupe ;
(ii) après lavage, mélanger le premier groupe de cellules avec un deuxième groupe de cellules de telle façon que des tubes membranaires intercellulaires se forment entre les cellules des premier et deuxième groupes après une deuxième durée ;
(iii) déterminer le nombre de cellules du deuxième groupe de cellules qui contiennent une ou plusieurs matières endocytées.

4. Procédé selon la revendication 3 dans lequel dans l'étape (i) le premier groupe de cellules est mélangé à une première matière endocytable et le deuxième groupe de cellules est mélangé à une deuxième matière endocytable, les première et deuxième substances étant des substances différentes.

5. Procédé selon la revendication 3 ou 4 dans lequel les substances endocytables sont choisies parmi colorants, colorants fluorescents, Dil, DiO, marqueurs radioactifs, colorants luminescents, protéines et peptides fluorescents ou luminescents, protéines ou peptides liés à des marqueurs.

6. Procédé selon l'une des revendications 2 à 5 dans lequel dans l'étape (i) l'endocytose de la substance dans une organelle est remplacée par une expression constitutive ou transitoire de la substance dans l'organelle.

7. Procédé selon l'une des revendications 2 à 6 dans lequel l'étape (iii) se fait par cytométrie en flux (FACS).

8. Procédé selon l'une des revendications 2 à 7 dans lequel l'étape (ii) se fait respectivement en présence ou sous l'influence d'un agent de contrôle.

9. Procédé selon la revendication 8 dans lequel l'agent de contrôle est un composé chimique ou une substance présumée pharmaceutiquement active.

10. Procédé selon la revendication 8 dans lequel l'agent de contrôle est un médicament ou un agent thérapeutique.

11. Procédé selon la revendication 8 dans lequel l'agent de contrôle est un dispositif physique.

12. Procédé selon la revendication 8 dans lequel l'agent de contrôle est un dispositif physique thérapeutique.

13. Procédé selon la revendication 1 dans lequel l'examen se fait par un système microscopique qui permet l'observation de différents niveaux microscopiques dans l'axe Z.

14. Procédé selon la revendication 13 dans lequel le système microscopique comprend un microscope, un régleur de niveau z et un système de commande correspondant.

15. Utilisation du procédé selon l'une des revendications 1 à 14 pour l'examen systématique de médicaments et agents actifs présumés.

16. Utilisation du procédé selon la revendication 15 pour l'examen systématique de médicaments et agents actifs présumés pour le traitement de tumeurs, d'hypertonie, de maladies infectieuses virales, bactériales ou parasitiques, de maladies du métabolisme, de maladies du système nerveux, de la psyché et de l'esprit, du niveau de cholestérol et dans le domaine de l'ingénierie tissulaire.

17. Utilisation du procédé selon la revendication 15 pour l'examen d'agents actifs en thérapie génique, en vectorisation de cellules, en pharmacologie et dans l'ingénierie tissulaire.
